# EUROPÄISCHE PATENTSCHRIFT

(11) **EP 1 859 730 B1**
(45) Veröffentlichungstag und Bekanntmachung des Hinweises auf die Patenterteilung: **28.07.2010**
(21) Anmeldenummer: 07009720.9
(22) Anmeldetag: 15.05.2007
(51) Int. Cl.: A61B 5/00

(54) **Sensor zur Messung eines Vitalparameters eines Lebewesens**
Sensor for measuring a vital parameter of a living organism
Capteur destiné à mesurer des signes vitaux d'un être vivant

(30) Priorität: 24.05.2006 DE 102006024459
(43) Veröffentlichungstag der Anmeldung: 28.11.2007
(73) Patentinhaber: Fraunhofer-Gesellschaft zur Förderung der angewandten Forschung e.V., 80686 München (DE)
(72) Erfinder: Couronné, Robert, 91058 Erlangen (DE); Bode, Stephan, 67433 Neustadt (DE); Aschenbrenner, Stefan, 90542 Eckental (DE); Mörsdorf, Hans-Joachim, 90763 Fürth (DE)
(74) Vertreter: Zimmermann, Tankred Klaus

(56) Entgegenhaltungen:
- EP-A- 1 151 719
- EP-A- 1 297 784
- EP-A- 1 374 763
- WO-A-99/60918
- WO-A-2006/038589
- WO-A-2007/038543
- WO-A2-01/52718
- WO-A2-98/17172
- US-A- 5 025 791
- US-A1- 2004 034 293
- US-A1- 2005 043 763
- US-A1- 2006 084 879

## Beschreibung

Die vorliegende Erfindung bezieht sich im Allgemeinen auf einen Sensor, eine Verarbeitungseinrichtung und ein Computerprogramm zum Liefern einer Information über einen Vitalparameter eines Lebewesens, im Speziellen auf eine bewegungsartefaktsensitive Transmissions-Plethysmographie am Handgelenk.

Im Bereich der Medizin ist es in vielen Situationen erforderlich, Vitalparameter eines Menschen bzw. Lebewesens zu erfassen. Es hat sich ferner gezeigt, dass es im Rahmen der zunehmenden Automatisierung wünschenswert ist, die Vitalparameter kontinuierlich in elektronischer Form erfassen zu können. Eine Möglichkeit zur Bestimmung von wichtigen Vitalparametern eines Menschens bzw. eines Lebewesens ist die Aufnahme eines Plethysmogramms. Ein Plethysmogramm ist eine graphische Abbildung von Volumenänderungen. In der Medizin wird ein Plethysmogramm unter anderem dazu verwendet, um Volumenänderungen arterieller Blutgefässe im menschlichen Körper darzustellen. Zur Aufnahme eines Plethysmogramms an einem Patienten wird typischerweise eine Sensorvorrichtung verwendet, die eine Lichtquelle und einen Photoempfänger enthält, und die so geartet ist, dass Licht die Gewebsschichten passiert und die verbleibende Lichtintensität von dem Photoempfänger gemessen wird. Passiert das Licht die Gewebsschichten, so erfährt es eine Dämpfung, die unter anderem abhängig von der Wellenlänge des Lichts, der Art und Konzentration der Stoffe in dem bestrahlten Gewebe und Volumenänderungen des arteriellen Blutstromes ist. Der Photoempfänger wandelt das auftreffende Licht in einen Photostrom, dessen Amplitude von den durch Herzmuskelkontraktionen verursachten Volumenänderungen der arteriellen Blutgefäße moduliert wird.

Bekannte Photoplethysmographen werden üblicherweise am Finger oder am Ohrläppchen des Patienten angebracht, weil dort die oberen Hautschichten sehr dicht mit arteriellen Blutgefäßen durchsetzt sind, und weil dort ferner der dämpfende Einfluss von Knochen oder Fettgewebe minimal ist. Zum Einsatz kommen sowohl auf dem Transmissionsprinzip als auch auf dem Remissionsprinzip basierende Plethysmographen.

Beim Remissionsverfahren wird der Finger nicht vollständig durchstrahlt, wie beim Transmissionsverfahren, sondern es wird nach Lichteinstrahlung der vom Gewebe emittierte Lichtanteil gemessen.

Allen Photoplethysmographen für den Einsatz am Finger, angebracht z. B. durch einen Fingerclip an der Fingerkuppe, ist eine Einschränkung der Bewegungsfreiheit des Patienten gemeinsam. Ferner wurde festgestellt, dass herkömmliche Plethysmographen in manchen Betriebsfällen unzuverlässige Werte liefern.

Die EP 1 297 784 A1 beschreibt ein Verfahren und eine Vorrichtung zur Pulsraten-Detektion. Eine Strahlungsenergie wird durch ein Gewebe eines menschlichen Körpers gestrahlt. Eine Intensität der Strahlungsenergie nach der Ausbreitung durch das Gewebe des menschlichen Körpers wird unter Verwendung von Lichtdetektoren gemessen. Eingangssignale, die diese Ausbreitung beschreiben, werden bereitgestellt. Eine Einrichtung zur Bewegungsdetektion liefert ein Bewegungs-Referenz-Signal, das eine Bewegung der Detektionseinrichtung im Hinblick auf das Gewebe des menschlichen Körpers beschreibt. Die Eingangssignale werden dann verarbeitet, um Bewegungs-bedingte Beiträge aufgrund der Bewegung der Detektionseinrichtung im Hinblick auf das Gewebe des menschlichen Körpers zu entfernen.

Die US 5,025,791 beschreibt ein Puls-Oximeter mit einem physikalischen Bewegungssensor. Ein Detektor umfasst einen oder mehrere Sensoren zur Detektion einer physikalischen Bewegung eines Subjekts, so dass Auslesewerte, die durch eine Bewegung des Subjekts beeinflusst sind, eliminiert oder angezeigt werden können.

Die WO 98/17172 beschreibt einen Finger-Ring-Sensor zur Patientenüberwachung. Das Überwachungssystem überwacht einen Gesundheits-Zustand eines Patienten und sendet ein Signal an einen entfernten Empfänger. Der Sensor ist in einem Finger-Ring eingebaut, um eine Hauttemperatur, einen Blut-Fluss, eine Konzentration von Blutbestandteilen oder eine Pulsrate des Patienten zu messen. Die Daten werden für eine drahtlose Übertragung codierte. Mehrere ringförmige Bänder oder Sensorelemente können eingesetzt werden, um dreidimensionale dynamische Eigenschaften von Arterien und Gewebe zu bestimmen.

Die EP 1 374 763 A1 beschreibt eine portable Einrichtung zur Messung und/oder Überwachung einer Herz-Rate. Die portable Einrichtung umfasst eine Kombination aus einer Herz-Raten-Messeinrichtung und einer Audio-Wiedergabe-Einheit.

Die Messeinrichtung und ein Schallwandler sind zumindest teilweise an einem Bauteil angebracht, das ausgelegt ist, um an einem Ohr eines Benutzers der Einrichtung angebracht zu werden. Die Audio-Wiedergabeeinheit umfasst eine Einrichtung zum Überlagern eines Signals, das die Herz-Rate darstellt, mit dem Audiosignal.

Die US 2006/0084879 A1 beschreibt eine Bewegungs-Kompensation von optischen Eingangssignalen für eine physiologische Pulsmessung. Ein Puls-Raten-Sensor umfasst einen Beschleunigungsmesser, um eine periodische Bewegung zu messen, und einen Piezo-Sensor, um eine unregelmäßige Bewegung zu detektieren. Der Pulsratensensor hilft, eine Pulsrate genauer zu bestimmen, indem die beiden Arten von Bewegungen berücksichtigt werden. Der Puls-Raten-Sensor kombiniert Algorithmen, die eine Signalverstärkung, eine Wellenformverfeinerung und eine Signal-Rausch-Unterdrückung steuern.

Die US 2004/0034293 A1 beschreibt ein Puls-Oximeter mit einer Bewegungsdetektion. Bewegungsbedingte Artefakte bei klinischen Überwachungsinstrumenten, die an einem Patienten angebracht sind, werden kompensiert. Bei einer Puls-Oximetrie werden zusätzliche Signale verwendet, um bei einer Triggerung des Puls-Oximeters oder bei einer Analyse der von dem Puls-Oximeter empfangenen Daten verwendet zu werden.

Die EP 1 151 719 A2 beschreibt eine Vorrichtung zur Überwachung von Herzfehlern unter Verwendung von Datenmustern.

Es ist daher die Aufgabe der vorliegenden Erfindung, ein Konzept zum Liefern von Informationen über einen Vitalparameter eines Lebewesens zu schaffen, das an einen Einsatz unter erschwerten Betriebsbedingungen angepasst ist.

Diese Aufgabe wird durch einen Sensor zum Liefern von Informationen über einen Vitalparameter gemäß Anspruch 1, eine Verarbeitungseinrichtung zum Liefern von Informationen über einen Vitalparameter gemäß Anspruch 23, ein Verfahren zum Liefern einer Information über einen Vitalparameter gemäß Anspruch 24 sowie ein Verfahren zum Liefern einer Information über einen Vitalparameter gemäß Anspruch 25 sowie durch ein Computerprogramm gemäß Anspruch 29 gelöst.

Die vorliegende Erfindung schafft einen Sensor zum Liefern von Informationen über einen Vitalparameter eines Lebewesens mit einer Befestigungseinrichtung zum Anbringen des Sensors an dem Lebewesen, einer Lichtquelle, die mit der Befestigungseinrichtung verbunden ist, um Licht in einen Körperteil des Lebewesens einzustrahlen, einem Lichtempfänger, der mit der Befestigungseinrichtung verbunden ist, und der ausgelegt ist, um einen Teil des eingestrahlten Lichts zu empfangen, um in Abhängigkeit von einer Intensität des empfangenen Lichts ein Lichtintensitäts-Signal zu liefern, das von dem Vitalparameter abhängt, und einem Beschleunigungssensor, der mit der Befestigungseinrichtung verbunden ist, und der ausgelegt ist, um in Abhängigkeit von einer Beschleunigung in zumindest einer Richtung ein Beschleunigungssignal zu liefern. Der Sensor ist ausgelegt, um das Lichtintensitätssignal und das Beschleunigungssignal an eine Verarbeitungseinrichtung zur verknüpfenden Verarbeitung des Lichtintensitätssignals und des Beschleunigungssignals zu übertragen, oder um das Lichtintensitätssignal in Abhängigkeit von dem Beschleunigungssignal zu erzeugen.

Es ist der Kerngedanke der vorliegenden Erfindung, dass die Zuverlässigkeit eines Sensors zum Liefern von Informationen über einen Vitalparameter, der eine durch einen Körperteil eines Lebewesens übertragene Lichtintensität auswertet, verbessert werden kann, indem eine Beschleunigung der Sensorenordnung durch einen Beschleunigungssensor ausgewertet wird. Es wurde nämlich herausgefunden, dass das von dem Lichtempfänger gelieferte Lichtintensitätssignal starken Schwankungen unterworfen ist, wenn auf die Sensorenordnung eine Beschleunigung einwirkt. Eine derartige Beschleunigung führt nämlich typischerweise dazu, dass sich eine relative Lage zwischen der Lichtquelle, dem Körperteil und dem Lichtempfänger verändert, und dass sich ferner durch die Beschleunigung auch innerhalb des Lebewesens Veränderungen ergeben, die einen Einfluss auf das Lichtintensitätssignal haben.

Es hat sich daher als vorteilhaft erwiesen, dass der Sensor das Lichtintensitätssignal und das Beschleunigungssignal gemeinsam an eine Verarbeitungseinrichtung zur verknüpfenden Verarbeitung des Lichtintensitätssignals und des Beschleunigungssignals überträgt. Durch die Verknüpfung des Lichtintensitätssignals und des Beschleunigungssignals kann dabei in der Verarbeitungseinrichtung erreicht werden, dass beschleunigungsbedingte Fehler in dem Lichtintensitätssignal bei der Ermittlung des Vitalparameters beispielsweise korrigiert werden, oder dass das Lichtintensitätssignal nicht für eine Berechnung des Vitalparameters herangezogen wird, falls der Beschleunigungssensor eine Beschleunigung feststellt, die außerhalb eines zulässigen Bereichs liegt. Alternativ dazu kann über die verknüpfenden Verarbeitung dem Lichtintensitätssignal eine von dem Beschleunigungssignal abhängige Zuverlässigkeitsinformation zugeordnet werden, die beispielsweise bei Vorliegen einer geringen Beschleunigung eine hohe Zuverlässigkeit des Lichtintensitätssignals anzeigt und umgekehrt.

Alternativ dazu hat es sich als vorteilhaft erwiesen, das Lichtintensitätssignal in Abhängigkeit von dem Beschleunigungssignal zu erzeugen, also beispielsweise bei Vorliegen einer Beschleunigung außerhalb eines zulässigen Bereichs entweder gar kein Lichtintensitätssignal zu erzeugen (beispielsweise durch Abschalten der Lichtquelle), oder ein korrigiertes Lichtintensitätssignal zu erzeugen.

In anderen Worten, es ist der Kerngedanke der vorliegenden Erfindung, bei dem Sensor zum Liefern der Informationen über den Vitalparameter entweder durch eine gemeinsame Bereitstellung des Lichtintensitätssignals und des Beschleunigungssignals für eine verknüpfende Verarbeitung eine Zuverlässigkeit einer ermittelten Informationen über den Vitalparameter zu erhöhen, oder alternativ das Lichtintensitätssignal in Abhängigkeit von dem Beschleunigungssignal zu bestimmen, und dabei einen Einfluss der Beschleunigung auf das Lichtintensitätssignal zu berücksichtigen.

Somit schafft die vorliegende Erfindung einen Sensor zum Liefern einer Information über einen Vitalparameter, der weniger empfindlich auf Erschütterungen reagiert als herkömmliche Sensoren, und der damit eine zuverlässige Aufnahmen und Auswertung eines Plethysmogramms erheblich erleichtert.

Ferner ermöglicht es die vorliegende Erfindung, eine Bewegungsfreiheit eines Menschen oder Lebewesens, an dem der Plethysmograph befestigt ist, gegenüber herkömmlichen Anordnungen zu erhöhen. Während nämlich bei bisherigen Plethysmographen die Patienten bzw. Menschen oder Lebewesen angehalten werden mussten, das Körperteil, an dem der Plethysmograph befestigt ist, nicht bzw. nur minimal zu bewegen, muss die Bewegungsfreiheit eines Menschen mit einem erfindungsgemäßen Sensor nicht wesentlich eingeschränkt werden. Durch die Verwendung eines Beschleunigungssensors und die Möglichkeit einer verknüpfenden Verarbeitung des Beschleunigungssignals und des Lichtintensitätssignals können Störungen des Lichtintensitätssignals durch Bewegungen entweder korrigiert oder zumindest erkannt werden. Damit wird es beispielsweise möglich, ein Plethysmogramm am Handgelenk eines Menschen aufzunehmen, ohne dass die Bewegungsfreiheit des Menschen wesentlich eingeschränkt wird, wobei dennoch eine zuverlässige Information über ein Vitalparameter des Menschen erhalten wird. Durch die Erkennung von Bewegungen ist dies selbst unter stark erschwerten Bedingungen bzw. Messbedingungen möglich, wie sie beispielsweise an dem Handgelenk herrschen. In anderen Worten, durch die Erkennung von Bewegungen bzw. Beschleunigungen kann beispielsweise der herkömmlicherweise störende Effekt, der durch das Vorhandensein von zueinander beweglichen Knochen am Handgelenk auftritt, im Wesentlichen kompensiert werden.

Somit ermöglicht es die vorliegende Erfindung insgesamt, auch unter erschwerten Bedingungen, wie beispielsweise bei Vorliegen von starken Bewegungen oder Beschleunigungen und selbst in Gegenwart von Knochen, die bei Bewegungen eine Relativbewegung zueinander aufweisen, mit hoher Zuverlässigkeit eine Information über einen Vitalparameter von einer Lichtübertragung bzw. von einer optischen Dämpfung zwischen einer Lichtquelle und einem Lichtempfänger abzuleiten.

Bei einem bevorzugten Ausführungsbeispiel umfasst der Vitalparameter eine Pulsfrequenz des Lebewesens. Die Lichtquelle und der Lichtempfänger sind dabei derart angeordnet, dass eine Lichtübertragung bzw. eine optische Dämpfung in dem Körperteil zwischen der Lichtquelle und dem Lichtempfänger durch eine Veränderung des Volumens eines Blutgefäßes in dem Körperteil beeinflusst wird. Die Bewegungs- bzw. Beschleunigungs-Abhängigkeit der optischen Dämpfung in dem Körperteil kann wiederum durch Verknüpfung des Lichtintensitätssignals mit dem Beschleunigungssignal kompensiert werden, oder es kann zumindest erkannt werden, wann das Lichtintensitätssignal aufgrund starker Bewegungen bzw. Beschleunigungen unzuverlässig ist.

Bei einem weiteren bevorzugten Ausführungsbeispiel umfasst der Vitalparameter einen Anteil verschiedener Blutbestandteile von Blut in einem Blutgefäß des Lebewesens, wobei die Anteile der verschiedenen Blutbestandteile des Bluts eine Wellenlängen-Abhängigkeit einer optischen Dämpfung in dem Körperteil zwischen der Lichtquelle und dem Lichtempfänger beeinflussen. Der Sensor ist in diesem Fall ausgelegt, um eine Wellenlängenabhängigkeit der optischen Dämpfung in dem Körperteil zwischen der Lichtquelle und den Lichtempfängern zu bestimmen.

Bei einem weiteren bevorzugten Ausführungsbeispiel sind die Lichtquelle und der Lichtempfänger angeordnet, um eine Transmissionsmessung durch das Körperteil zu ermöglichen. Alternativ können die Lichtquelle und der Lichtempfänger aber auch angeordnet sein, um einer Remissionsmessung durch das Körperteil zu ermöglichen.

Bei einem weiteren bevorzugten Ausführungsbeispiel ist die Befestigungseinrichtung ausgelegt, um den Sensor um eine menschliche Handwurzel, ein menschliches Handgelenk oder einen menschlichen Unterarm anzubringen. Es hat sich nämlich gezeigt, dass gerade in den genannten Bereichen Vitalparameter des Lebewesens besonders gut aus der optischen Dämpfung zwischen der Lichtquelle und dem Lichtempfänger bestimmt werden können, ohne eine unnötig große Einschränkung der Bewegungsfreiheit für das Lebewesen bzw. für den Menschen zu verursachen. Die im Bereich der Handwurzel, des Handgelenks oder des Unterarms auftretende Bewegungen können vorteilhafterweise durch den Beschleunigungssensor erfasst werden, so dass eine Kompensation der durch die Bewegung hervorgehobenen Bewegungsartefakte möglich ist. Die Befestigungseinrichtung kann beispielsweise ein starres oder flexibles Armband umfassen, dessen Größe ausgelegt ist, um an einer menschlichen Handwurzel, einem menschlichen Handgelenk oder einem menschlichen Unterarm angebracht zu werden.

Bei einem weiteren bevorzugten Ausführungsbeispiel ist die Befestigungseinrichtung ausgelegt, um den Sensor um ein menschliches Handgelenk anzubringen. In diesem Fall ist die Lichtquelle bevorzugt an der Befestigungseinrichtung angebracht, um von einer Außenseite des Handgelenks Licht in das Handgelenk einzustrahlen. Der Lichtempfänger ist in diesem Fall weiterhin bevorzugt an der Befestigungseinrichtung angebracht, um Licht von einer Innenseite des Handgelenks zu empfangen. Es hat sich nämlich gezeigt, dass eine Erfassung der Vitalparameter besonders vorteilhaft dadurch möglich ist, dass das Handgelenk von seiner Außenseite her zu seiner Innenseite hin von Licht durchstrahlt wird. Bei einer derartigen Anordnung von Lichtquelle und Lichtempfängern ist gewährleistet, dass eine Lichtausbreitung durch das Gelenk hindurch derart erfolgt, dass die Intensität des von dem Lichtempfänger empfangene Lichts maximiert' ist. Ferner stellt ein um das Handgelenk getragener Sensor für einen menschlichen Patienten typischerweise eine geringe Einschränkung dar, wobei der Sensor von seinen Trageeigenschaften her im Wesentlichen einer Armbanduhr entspricht.

Im Übrigen wird es bevorzugt, dass die Befestigungseinrichtung, die Lichtquelle und der Lichtempfänger ausgelegt sind, um den Sensor so an einem Körperteil oder um ein Körperteil zu befestigen, dass eine in dem Körperteil vorhandene Arterie zwischen der Lichtquelle und dem Lichtempfänger angeordnet ist. Durch die genannte Anordnung wird erreicht, dass die von dem Lichtempfänger empfangene Lichtintensität in einer maximalen Weise von dem Zustand der Arterie, also beispielsweise von dem Volumen der Arterie und der Beschaffenheit des in der Arterie vorhandenen Blutes abhängt. Somit wird eine maximale Empfindlichkeit des erfindungsgemäßen Sensors gewährleistet.

Bei einem weiteren bevorzugten Ausführungsbeispiel umfasst der Sensor als Verarbeitungseinrichtung eine Pulsbestimmungseinrichtung, die ausgelegt ist, um aus zeitlichen Schwankungen des Lichtintensitätssignals ein Pulsfrequenz des Lebewesens zu bestimmen, wobei die Pulsfrequenz des Lebewesens den Vitalparameter bildet. In anderen Worten, bei einem bevorzugten Ausführungsbeispiel ist die Verarbeitungseinrichtung zur verknüpfenden Verarbeitung des Lichtintensitätssignals und des Beschleunigungssignals an dem Sensor selbst angebracht, oder wird zumindest als ein Teil desselben betrachtet. Die Verarbeitungseinrichtung ist dabei ausgelegt, um das Lichtintensitätssignal und das Beschleunigungssignals gemeinsam zu verarbeiten bzw. zu verknüpfen, um einen Einfluss der Beschleunigung auf das Lichtintensitätssignal entweder zu kompensieren, oder um ein verknüpftes Signal zu erzeugen, das korrespondierende Informationen über das Lichtintensitätssignal und/oder den Vitalparameter sowie zusätzlich über eine Zuverlässigkeit des Lichtintensitätssignals und/oder des Vitalparameters umfasst. In anderen Worte, die Verarbeitungseinrichtung kann ausgelegt sein, um eine Folge von Informationspaaren zu liefern, wobei jedes Informationspaar eine Information über den Vitalparameter sowie eine zugeordnete Information über die Zuverlässigkeit des Vitalparameters umfasst, wobei die Information über die Zuverlässigkeit des Vitalparameters basierend auf dem Beschleunigungssignal bestimmt ist.

Alternativ dazu kann die Verarbeitungseinrichtung ausgelegt sein, um die Information über den Vitalparameter nur dann zu liefern, wenn das Beschleunigungssignal in einem vorgegebenen zulässigen Bereich ist, und um andernfalls eine Fehlerinformation zu liefern.

Bei einem weiteren bevorzugten Ausführungsbeispiel umfasst der Sensor eine Mehrzahl von Lichtquellen unterschiedlicher Lichtwellenlänge, und ist ausgelegt, um Licht unterschiedlicher Wellenlängen in das Körperteil einzustrahlen. Der Sensor ist in diesem Fall ferner bevorzugt ausgelegt, um eine optische Dämpfung zwischen den Lichtquellen und dem Lichtempfänger in Abhängigkeit von der Wellenlänge zu bestimmen.

Bei einem weiteren bevorzugten Ausführungsbeispiel umfasst der Sensor eine Mehrzahl von Lichtempfängern unterschiedlicher spektraler Empfindlichkeit, und ist angelegt, um eine Bestimmung einer optischen Dämpfung zwischen der Lichtquelle und den Lichtempfängern in Abhängigkeit von der Wellenlänge zu ermöglichen. In beiden Fällen ist es möglich, aus der Abhängigkeit der optischen Dämpfung von der Wellenlänge des Lichtes auf eine Zusammensetzung von Blut in einer Arterie zwischen der Lichtquelle und dem Lichtempfänger zu schließen.

Bei einem weiteren bevorzugten Ausführungsbeispiel umfasst der Sensor als Verarbeitungseinrichtung eine Blutzusammensetzungs-Bestimmungseinrichtung, die ausgelegt ist, um aus der Wellenlängen-Abhängigkeit der optischen Dämpfung in dem Körperteil zwischen der Lichtquelle und dem Lichtempfänger Anteile von verschiedenen Blutbestandteilen von Blut in einem Blutgefäß des Lebewesens zu bestimmen. Die Verarbeitungseinrichtung ist in diesem Fall ausgelegt, um das Lichtintensitätssignal und das Beschleunigungssignal zu kombinieren oder zu verknüpfen, um die Anteile der verschiedenen Blutbestandteile in Abhängigkeit sowohl von dem Lichtintensitätssignal als auch von dem Beschleunigungssignal zu bestimmen. Das Beschleunigungssignal kann für eine Korrektur oder für eine Bestimmung der Zuverlässigkeit der bestimmten Werte herangezogen werden, wie dies schon oben erläutert wurde.

Bei einem bevorzugten Ausführungsbeispiel umfasst der Sensor die Verarbeitungseinrichtung, und die Verarbeitungseinrichtung ist ausgelegt, um das Lichtintensitätssignal in Abhängigkeit von dem Beschleunigungssignal zu korrigieren, um Veränderungen des Lichtintensitätssignals aufgrund der Beschleunigung entgegenzuwirken. Somit kann selbst bei Vorliegen einer Beschleunigung noch ein zuverlässiges Lichtintensitätssignal erhalten werden, das eine tatsächliche, um die Beschleunigung bzw. beschleunigungsbedingte Effekte korrigierte, optische Dämpfung in dem Körperteil zwischen der Lichtquelle und dem Lichtempfänger beschreibt.

Bei einem weiteren bevorzugten Ausführungsbeispiel umfasst der Sensor die Verarbeitungseinrichtung, wobei die Verarbeitungseinrichtung ausgelegt ist, um die Information über den Vitalparameter aus dem Lichtintensitätssignal zu bestimmen, und wobei die Verarbeitungseinrichtung ferner ausgelegt ist, um die Information über den Vitalparameter in Abhängigkeit von dem Beschleunigungssignal zu korrigieren, um einem Fehler der Information über den Vitalparameter aufgrund der Beschleunigung entgegenzuwirken. In anderen Worten, die Verarbeitungseinrichtung kann ein durch die Beschleunigung beeinflusstes Lichtintensitätssignal empfangen und das Beschleunigungssignal dann bei einer Berechnung des Vitalparameters aus dem Lichtintensitätssignal einfließen lassen.

In anderen Worten es bestehen verschiedene Möglichkeiten, an welcher Stelle der Verarbeitungskette das Beschleunigungssignal einwirkt. So kann bei einem Ausführungsbeispiel der vorliegenden Erfindung das Beschleunigungssignal dazu verwendet werden, um das Lichtintensitätssignal zu korrigieren, und um somit auch bei Einwirken einer Beschleunigung auf den Sensor ein Lichtintensitätssignal zu erhalten, das einem Lichtintensitätssignal ohne Einwirken der Beschleunigung entspricht. Bei einem anderen Ausführungsbeispiel wird bei Einwirkung einer Beschleunigung zwar ein verfälschtes Lichtintensitätssignal erzeugt, der Einfluss der Beschleunigung wird allerdings bei der Bestimmung der Informationen über den Vitalparameter eliminiert oder minimiert, indem die Einrichtung zum Ableiten der Informationen über den Vitalparameter aus dem Lichtintensitätssignal das Beschleunigungssignal empfängt, und in Abhängigkeit von dem Beschleunigungssignal den Algorithmus zur Bestimmung der Information über den Vitalparameter aus dem Lichtintensitätssignal anpasst (z.B. durch Beschleunigungsabhängige Veränderung von Parametern, oder durch lineare oder nicht-lineare Verknüpfung von bei der Bestimmung der Information über den Vitalparameter auftretenden Signalen mit dem Beschleunigungssignal.

Bei einem weiteren bevorzugten Ausführungsbeispiel umfasst der Sensor die Verarbeitungseinrichtung, wobei die Verarbeitungseinrichtung ausgelegt ist, um die Information über den Vitalparameter aus dem Lichtintensitätssignal zu bestimmen. Bei dem genannten Ausführungsbeispiel ist die Verarbeitungseinrichtung ferner ausgelegt, um aus dem Beschleunigungssignal eine der Informationen über den Vitalparameter zugeordnete Zuverlässigkeitsinformation zu erzeugen, die bei Vorliegen der betragsmäßig kleinen Beschleunigung eine hohe Zuverlässigkeit der Informationen über den Vitalparameter anzeigt, und die bei Vorliegen einer betragsmäßig größeren Beschleunigung eine geringere Zuverlässigkeit der Information über den Vitalparameter anzeigt. Somit wird beispielsweise unabhängig von der Beschleunigung stets eine Information über den Vitalparameter berechnet, jedoch zusätzlich zu der Information über den Vitalparameter eine Information über die Zulässigkeit derselben bestimmt. Bei einer weiteren Verarbeitung der Information über den Vitalparameter kann diese Zuverlässigkeitsinformation beispielsweise mit berücksichtigt werden. Wird beispielsweise ein Mittelwert (z. B. ein zeitlicher Mittelwert) über die Information über den Vitalparameter gebildet, so kann die Zuverlässigkeitsinformation verwendet werden, um eine Gewichtung durchzuführen, um also beispielsweise bei der Bildung eines gewichteten Mittelwerts der Information über den Vitalparameter ein hohes Gewicht zuzuordnen, wenn die Information über den Vitalparameter aufgrund der Zuverlässigkeitsinformation als zuverlässig angesehen wird. Umgekehrt kann der Information über den Vitalparameter ein geringes Gewicht zugeordnet werden, wenn die Information über den Vitalparameter als wenig zuverlässig angesehen wird.

Bei einem weiteren bevorzugten Ausführungsbeispiel umfasst der Sensor eine Verarbeitungseinrichtung, die ausgelegt ist, um die Information über den Vitalparameter aus dem Lichtintensitätssignal nur dann zu bestimmen, wenn das Beschleunigungssignal anzeigt, dass die Beschleunigung innerhalb eines vorgegebenen zulässigen Bereichs liegt, und um andernfalls anstelle der Information über den Vitalparameter eine früher bestimmte Information über den Vitalparameter oder ein Fehlersignal, das einen Fehler anzeigt, zu liefern, oder keine Information über den Vitalparameter zu liefern. In anderen Worten, wird festgestellt, dass die Beschleunigung außerhalb des zulässigen Bereichs liegt, und kann in diesem Fall daher keine zuverlässige Information über einen Vitalparameter bestimmt werden, so hat es sich als vorteilhaft erwiesen, beispielsweise eine vorher bestimmte Information über den Vitalparameter noch einmal auszugeben. Somit werden in einer kontinuierlichen Folge die Informationen über den Vitalparameter ausgegeben, wobei zu Zeiten, zu denen eine starke Beschleunigung auftritt, keine Aktualisierung der Informationen über den Vitalparameter stattfindet, sondern vielmehr ein vorher bestimmter Vitalparameter weiter ausgegeben wird. Diese Funktionalität basiert auf der Erkenntnis, dass sich typischerweise der Vitalparameter während der vergleichsweise kurzen Zeitintervalle, während denen eine starke Beschleunigung auftritt, nicht wesentlich verändert. Im Übrigen hat sich ferner gezeigt, dass bei typischen Bewegungsabläufen eines Patienten bzw. Menschen oder Lebewesens in ausreichender Regelmäßigkeit Zustände auftreten, während derer die Beschleunigung hinreichend klein ist, so dass ein Vitalparameter mit ausreichender Häufigkeit zuverlässig bestimmt werden kann. Somit liefert die erfindungsgemäßen Vorrichtung bei der genannten Ausgestaltung eine kontinuierliche Folge von Informationen über den Vitalparameter, wobei Änderungen des Vitalparameters ausreichend schnell erkannt werden, und wobei somit für jeden Zeitpunkt eine zuverlässige der Information über den Vitalparameter ausgegeben wird. Alternativ dazu kann der Sensor während Zeitintervallen, in denen eine unzulässig hohe Beschleunigung auftritt, auch ein Fehlersignal und/oder keine Information über den Vitalparameter liefern. Auf diese Weise kann wirksam verhindert werden, dass ein mit dem Sensor gekoppeltes Auswertegerät eine unzuverlässige Information über den Vitalparameter empfängt.

Bei einem weiteren bevorzugten Ausführungsbeispiel umfasst der Sensor ferner eine Dämpfungsmaß-Erfassungseinrichtung, die ausgelegt ist, um eine Dämpfungsinformation, die eine optische Dämpfung zwischen der Lichtquelle und dem Lichtempfänger beschreibt, zu ermitteln, sowie eine Lichtquellen-Einstellungseinrichtung, die ausgelegt ist, um eine von der Lichtquelle eingestrahlte Lichtleistung in Abhängigkeit von der Dämpfungsinformation einzustellen. In anderen Worten, die Lichtquellen-Einstellungseinrichtung empfängt die optische Dämpfung zwischen der Lichtquelle und dem Lichtempfänger, und regelt die von der Lichtquelle in den Körperteil eingestrahlte Lichtleistung derart, dass der Lichtempfänger eine für einen zuverlässigen Betrieb ausreichende Lichtleistung empfängt. Durch die erfindungsgemäße Erfassung des Dämpfungsmaßes wird erreicht, dass die Intensität der Lichtquelle auf einen optimalen Wert eingestellt werden kann. Es wird somit vermieden, dass die Lichtquelle eine zu hohe Lichtleistung abstrahlt, was unter anderem in einer unnötig hohen Leistungsaufnahme und folglich einer unnötig kurzen Batterielaufzeit resultieren würde. Andererseits wird auch vermieden, dass die Lichtquelle eine zu geringe Lichtleistung abstrahlt, was in einer Unzuverlässigkeit des von dem Lichtempfänger gelieferten Lichtintensitätssignals resultieren würde.

Bei einem weiteren bevorzugten Ausführungsbeispiel ist die Dämpfungsmaß-Erfassungseinrichtung ausgelegt, um eine Information über einen Wasseranteil in einem Gewebe eines Körperteils zu bestimmen, und um die Dämpfungsinformation von der Information über den Wasseranteil abzuleiten. Es hat sich nämlich gezeigt, dass der Wasseranteil in dem Gewebe einen starken Einfluss auf die optische Dämpfung zwischen der Lichtquelle und dem Lichtempfänger ausübt. Somit wird die Lichtintensität in Abhängigkeit von einer erwarteten optischen Dämpfung zwischen der Lichtquelle und dem Lichtempfänger eingestellt. Es sei hierbei ferner darauf hingewiesen, dass die Einstellung der von der Lichtquelle abgestrahlten Lichtleistung basierend auf der Information über den Wasseranteil in dem Gewebe gegenüber einer optischen Regelung (beispielsweise basierend auf dem Lichtintensitätssignal als Regelgröße) den wesentlichen Vorteil mit sich bringt, das keine aufwändige Filterung des Lichtintensitätssignals erforderlich ist. Gerade Schwankungen des Lichtintensitätssignals stellen nämlich eine Nutzinformation dar, die freilich nicht zu Null ausgeregelt werden darf. Im Übrigen ist die Bestimmung des Wasseranteils in dem Gewebe unabhängig von Störeffekten wie beispielsweise einen vorübergehenden Vorhandensein von Knochen zwischen der Lichtquelle und dem Lichtempfänger. Zusammenfassend lässt sich somit festhalten, dass die Einstellung der von der Lichtquelle eingestrahlten Lichtleistung in Abhängigkeit von der Information über den Wasseranteil in dem Gewebe eine besonders zuverlässige Einstellung der Lichtintensität gewährleistet, die von störenden Effekten wie Beschleunigungen, dem Vorhandensein von Knochen, dem Volumen von Blutgefäßen sowie der Konsistenz des Blutes besonders wenig beeinflusst wird.

Bei einem weiteren bevorzugten Ausführungsbeispiel ist die Dämpfungsmaß-Erfassungseinrichtung ausgelegt, um eine Hautimpedanz des Körperteils zu bestimmen, und um die Dämpfungsinformationen von der Hautimpedanz abzuleiten. Es hat sich nämlich gezeigt, dass die Hautimpedanz, also eine Impedanz zwischen zwei Elektroden, die mit verschiedenen Stellen der Haut in Kontakt stehen, eine zuverlässige Aussage über den Wassergehalt des Gewebes und damit über die Dämpfungseigenschaften des Körperteils liefert.

Die vorliegende Erfindung umfasst ferner eine Verarbeitungseinrichtung zum Liefern von Informationen über einen Vitalparameter eines Lebewesens basierend auf einem Lichtintensitätssignal und einem Beschleunigungssignal von einem Sensor, wobei das Lichtintensitätssignal eine Intensität eines von einem an dem Lebewesen angebrachten Lichtempfänger aus einem Körperteil des Lebewesens empfangenen Lichts beschreibt, und wobei das Beschleunigungssignal eine Beschleunigung am Ort eines mit dem Lichtempfänger verbundenen Beschleunigungssensors beschreibt. Die Verarbeitungseinrichtung umfasst eine Einrichtung, die ausgelegt ist, um das Lichtintensitätssignal und das Beschleunigungssignal zu verknüpfen, um den Vitalparameter zu ermitteln. In anderen Worten, die Verarbeitungseinrichtung kann das Lichtintensitätssignal basierend auf dem Beschleunigungssignal korrigieren, eine kombinierte Information erzeugen, die sowohl das Lichtintensitätssignal als auch das Beschleunigungssignal umfasst, oder basierend auf dem Beschleunigungssignal die Erzeugung des Lichtintensitätssignals unterbinden.

Im Übrigen sein darauf hingewiesen, dass die Verarbeitungseinrichtung um all diejenigen Merkmale ergänzt werden können, die bereits im Hinblick auf die zu dem Sensor gehörige Verarbeitungseinrichtung beschrieben worden sind.

Ferner umfasst die vorliegende Erfindung ein Verfahren zum Liefern einer Information über einen Vitalparameter eines Lebewesens. Das Verfahren umfasst ein Bestimmen einer Information über eine optische Dämpfung in einem Körperteil des Lebewesens zwischen einer Lichtquelle und einem Lichtempfänger, wobei die optische Dämpfung von dem Vitalparameter abhängt, und wobei die Lichtquelle und der Lichtempfänger an dem Körperteil mit einer Befestigungseinrichtung angebracht sind. Das Verfahren umfasst ferner das Bestimmen einer Information über eine Beschleunigung der Lichtquelle, des Lichtempfängers oder der Befestigungseinrichtung, sowie ein Verknüpfen der Information über die optische Dämpfung mit der Information über die Beschleunigung, um die Information über den Vitalparameter zu erhalten.

Die vorliegende Erfindung umfasst ferner ein Verfahren zum Liefern einer Information über einen Vitalparameter eines Lebewesens in einer Einrichtung mit einer Lichtquelle und einem Lichtempfänger, die angeordnet sind, um eine Information über eine optische Dämpfung in einem Körperteil des Lebewesens zwischen der Lichtquelle und dem Lichtempfänger zu bestimmen, wobei die optische Dämpfung von dem Vitalparameter abhängt, und wobei die Lichtquelle und der Lichtempfänger an dem Körperteil mit einer Befestigungseinrichtung angebracht sind. Das Verfahren umfasst ein Bestimmen einer Information über eine Beschleunigung der Lichtquelle, dem Lichtempfängers oder der Befestigungseinrichtung. Das Verfahren umfasst ferner ein Abschalten der Lichtquelle und/oder ein Einstellen bzw. Unterbrechen der Erzeugung der Information über den Vitalparameter, falls die Information über die Beschleunigung anzeigt, dass die Beschleunigung größer als eine vorgegebene maximal zulässige Beschleunigung ist.

Die vorliegende Erfindung umfasst ferner ein Computerprogramm zur Realisierung der erfindungsgemäßen Verfahren.

Bevorzugte Ausführungsbeispiele der vorliegenden Erfindung werden nachfolgend Bezug nehmend auf die beiliegenden Figuren näher erläutert. Es zeigen:
- Fig. 1A: eine schematische Darstellung eines erfindungsgemäßen Sensors zum Liefern einer Information über einen Vitalparameter eines Lebewesens gemäß einem ersten Ausführungsbeispiel der vorliegenden Erfindung;
- Fig. 1B: eine schematische Darstellung eines erfindungsgemäßen Sensors zum Liefern eines Vitalparameters eines Lebewesens gemäß einem zweiten Ausführungsbeispiel der vorliegenden Erfindung;
- Fig. 2A: eine schematische Darstellung einer erfindungsgemäßen Verarbeitungseinrichtung zum verknüpfenden Verarbeiten eines Lichtintensitätssignals und eines Beschleunigungssignals, gemäß einem dritten Ausführungsbeispiel der vorliegenden Erfindung;
- Fig. 2B: eine schematische Darstellung einer erfindungsgemäßen Verarbeitungseinrichtung zum verknüpfenden Verarbeiten eines Lichtintensitätssignals und eines Beschleunigungssignals, gemäß einem vierten Ausführungsbeispiel der vorliegenden Erfindung;
- Fig. 2C: eine schematische Darstellung einer erfindungsgemäßen Verarbeitungseinrichtung zum verknüpfenden Verarbeiten eines Lichtintensitätssignals und eines Beschleunigungssignals gemäß einem fünften Ausführungsbeispiel der vorliegenden Erfindung;
- Fig. 3A: eine Querschnittsdarstellung eines erfindungsgemäßen Sensors, der um einen menschlichen Unterarm befestigt ist;
- Fig. 3B: ein Schrägbild eines erfindungsgemäßen Sensors, der um einen menschlichen Unterarm befestigt ist;
- Fig. 4: eine schematische Darstellung eines erfindungsgemäßen Sensors einschließlich einer Schaltungsanordnung zur Ansteuerung der Lichtquelle und einer Schaltungsanordnung zur Auswertung des Lichtintensitätssignals;
- Fig. 5: ein Blockschaltbild einer erfindungsgemäßen Schaltungsanordnungseinstellung zur Einstellung einer von einer Lichtquelle abgegebenen Lichtmenge basierend auf einer Hautimpedanz, gemäß einem weiteren Ausführungsbeispiel der vorliegenden Erfindung;
- Fig. 6: ein Blockschaltbild eines erfindungsgemäßen Verfahrens, gemäß einem weiteren Ausführungsbeispiel der vorliegenden Erfindung; und
- Fig. 7: ein Blockschaltbild eines erfindungsgemäßen Verfahrens, gemäß einem weiteren Ausführungsbeispiel der vorliegenden Erfindung;

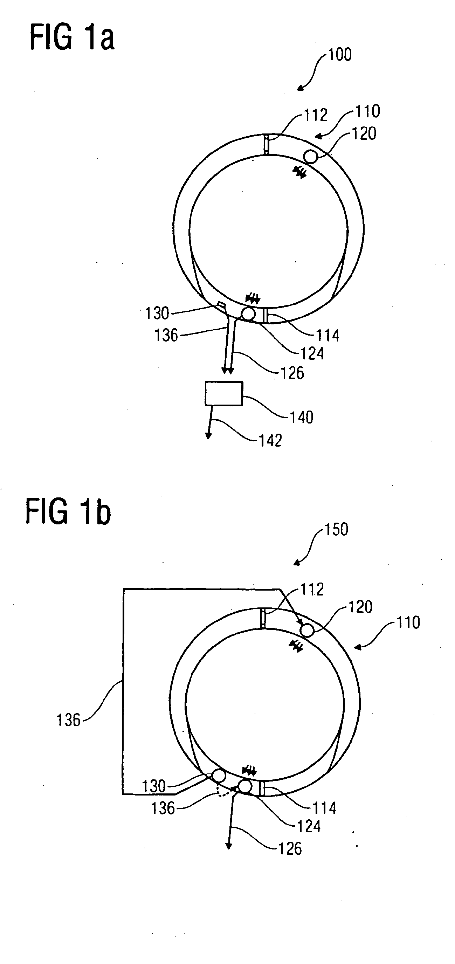

Fig. 1A zeigt eine schematische Darstellung eines erfindungsgemäßen Sensors zum Liefern einer Information über einen Vitalparameter eines Lebewesens, gemäß einem ersten Ausführungsbeispiel der vorliegenden Erfindung. Der Sensor gemäß der Fig. 1A ist in seiner Gesamtheit mit 100 bezeichnet. Der Sensor umfasst eine Befestigungseinrichtung 110 zum Anbringen des Sensors an dem Lebewesen. Bei der Befestigungseinrichtung 110 kann es sich beispielsweise um einen Armring mit einem Scharnier 112 und einen Verschluss 114 handeln. Alternativ dazu kann die Befestigungseinrichtung 110 auch als ein Armband ausgeführt sein, wie es beispielsweise in Verbindung mit Armbanduhren eingesetzt wird. Die Befestigungseinrichtung 110 kann ferner einteilig aus einem elastischen Material gefertigt sein, und kann ausgelegt sein, um an einem Stück an einem Körperteil des Lebewesens befestigt zu werden. Die Befestigungseinrichtung 110 kann ferner beispielsweise aus einem Metall gefertigt sein.

Alternativ dazu kann die Befestigungseinrichtung 110 aus Kunststoff gefertigt sein. An der Befestigungseinrichtung 110 ist eine Lichtquelle 120 befestigt, die ausgelegt und/oder angeordnet ist, um Licht in einen Körperteil des Lebewesens einzustrahlen. Handelt es sich beispielsweise bei der Befestigungseinrichtung 110 um ein Armband oder einen Armring, der ausgelegt ist, um um eine menschliche Handwurzel, ein menschliches Handgelenk oder einen menschlichen Arm befestigt zu werden, so ist die Lichtquelle 120 bevorzugt angeordnet, um Licht in die Handwurzel, das Handgelenk oder den Arm hinein abzustrahlen. In anderen Worten, handelt es sich beispielsweise bei der Befestigungseinrichtung 110 um einen Armring oder ein Armband, so ist die Lichtquelle 120 bevorzugt angeordnet, um Licht zu der Innenseite des Armbandes oder Armrings hin abzustrahlen.

Ganz allgemein lässt sich also feststellen, dass die Lichtquelle 120 ausgelegt ist, um Licht in das Körperteil einzustrahlen, das von der Befestigungseinrichtung 110 zumindest teilweise umschlossen wird.

An der Befestigungseinrichtung 110 ist ferner ein Lichtempfänger 124 angebracht. Der Lichtempfänger 124 ist ausgelegt, um einen Teil des in den Körperteil eingestrahlten Lichts zu empfangen, und um in Abhängigkeit von einer Intensität des empfangenen Lichts ein Lichtintensitätssignal 126 zu liefern, das von dem Vitalparameter abhängt. Zu diesem Zweck ist der Lichtempfänger 124 bevorzugt so an der Befestigungseinrichtung 110 angebracht, das eine Richtung maximaler Empfindlichkeit des Lichtempfängers 124 zu der Innenseite der Befestigungseinrichtung 110 hin bzw. zu einem Körperteil hin, das von der Befestigungseinrichtung 110 zumindest teilweise umschlossen wird, orientiert ist. Handelt es sich bei der Befestigungseinrichtung 110 um einen Armring oder ein Armband, so ist der Lichtempfänger 124 bevorzugt an der Innenseite des Armrings oder Armbandes angeordnet, oder zumindest ausgelegt, um Licht von der Innenseite des Armrings oder Armbands her empfangen zu können.

Der Sensor 100 umfasst ferner einen Beschleunigungssensor 130, der mit der Befestigungseinrichtung 110 verbunden ist. Der Beschleunigungssensor ist ausgelegt, um in Abhängigkeit von einer Beschleunigung in zumindest einer Richtung ein Beschleunigungssignal 136 zu liefern. Der Beschleunigungssensor 130 ist somit mechanisch mit der Befestigungseinrichtung 110 gekoppelt, und erfährt im Wesentlichen die gleiche Beschleunigung wie die Befestigungseinrichtung 110. Ferner ist der Beschleunigungssensor 130 bevorzugt über die Befestigungseinrichtung 110 mechanisch mit dem Lichtempfänger 124 gekoppelt, und erfährt aus diesem Grund zumindest bei Vorliegen einer Beschleunigung in eine bestimmte Richtung zumindest näherungsweise dieselbe Beschleunigung wie der Lichtempfänger 124. Ferner ist der Beschleunigungssensor 130 bevorzugt mechanisch mit der Lichtquelle 120 gekoppelt, so dass Bewegungen der Lichtquelle 120 typischerweise durch eine in dem Beschleunigungssensor 130 auftretende Beschleunigung detektiert werden können.

Es sei hierbei freilich darauf hingewiesen, dass an dem Ort des Beschleunigungssensors 130 nicht exakt die gleichen Beschleunigungen auftreten müssen wie am Ort der Lichtquelle 120 oder des Lichtempfängers 124. Andererseits wirken sich jedoch eine Vielzahl von Bewegungen der Befestigungseinrichtung 110 zumindest in ähnlicher Weise auf die Lichtquelle 120, den Lichtempfänger 124 und den Beschleunigungssensor 130 aus, so dass bei einer Vielzahl von möglichen Bewegungen die am Ort des Beschleunigungssensors 130 auftretende Beschleunigung ein Maß für die Stärke einer Bewegung der Befestigungseinrichtung 110 bzw. der Lichtquelle 120 und/oder des Lichtempfängers 124 ist.

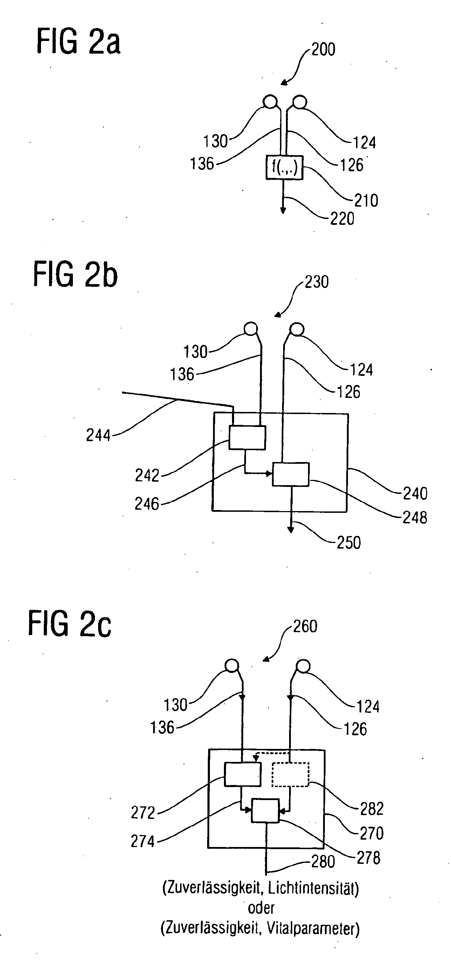

Der erfindungsgemäße Sensor 100 ist ferner ausgelegt, um das Lichtintensitätssignal 126 und das Beschleunigungssignal 136 an eine Verarbeitungseinrichtung 140 zur verknüpfenden Verarbeitung des Lichtintensitätssignals 126 und des Beschleunigungssignals 136 zu übertragen. In anderen Worten, der Sensor ist bevorzugt ausgelegt, um das Lichtintensitätssignal 126 und das Beschleunigungssignal 136 zeitlich koordiniert zu einer einzigen Verarbeitungseinrichtung 140 zu übertragen. Die Verarbeitungseinrichtung 140 zur verknüpfenden Verarbeitung des Lichtintensitätssignal 126 und des Beschleunigungssignals 126 kann optional ferner ein Teil des Sensors 100 sein, und kann beispielsweise ausgelegt sein, um eine Information 142 über den Vitalparameter zu liefern. Details im Hinblick auf die mögliche innere Struktur der Verarbeitungseinrichtung 140 werden beispielsweise mit Bezug auf die Fig. 2A, 2B und 2C beschrieben.

Der erfindungsgemäße Sensor 100 ermöglicht somit eine gemeinsame bzw. verknüpfende Verarbeitung des Lichtintensitätssignals 126 sowie des Beschleunigungssignals 136, wodurch die Zuverlässigkeit der durch die Verarbeitungseinrichtung 140 erzeugten Informationen 142 über den Vitalparameter gegenüber herkömmlichen Sensoren verbessert werden kann. Einflüsse der durch den Beschleunigungssensor 130 ermittelten Beschleunigung auf das Lichtintensitätssignal 126 oder auf die Information 142 über den Vitalparameter können minimiert werden.

Im Übrigen sein darauf hingewiesen, dass bei einem bevorzugten Ausführungsbeispiel die Lichtquelle 120 und der Lichtempfänger 124 ausgelegt bzw. angeordnet sind, um eine Transmissionsmessung durch ein Körperteil, das von der Befestigungseinrichtung 110 zumindest teilweise umschlossen wird, zu ermöglichen. In anderen Worten, die Lichtquelle 120 und der Lichtempfänger 124 sind bevorzugt so angeordnet bzw. ausgerichtet, dass die Lichtquelle 120 eine maximale Lichtintensität in die Richtung zu dem Lichtempfänger 124 hin ausstrahlt, und dass der Lichtempfänger 124 eine maximale Empfindlichkeit in der Richtung hin zu der Lichtquelle 120 aufweist.

Bei einem alternativen Ausführungsbeispiel können die Lichtquelle 120 und der Lichtempfänger 124 aber auch für eine Remissionsmessung ausgerichtet bzw. angeordnet sein, so dass von der Lichtquelle 120 ausgehendes Licht in dem Körperteil zu dem Lichtempfänger 124 hin gestreut bzw. reflektiert wird.

Weiterhin wird es im Übringen bevorzugt, dass die Lichtquelle 120 und der Lichtempfänger 124 so angeordnet sind, dass zwischen der Lichtquelle 120 und dem Lichtempfänger 124 eine Arterie des Lebewesens liegt, wenn die Befestigungseinrichtung an dem Lebewesen angebracht ist. Die Arterie kann dabei bevorzugt auf einer Verbindungslinie zwischen der Lichtquelle 120 und dem Lichtempfänger 124 liegen, oder die Arterie kann alternativ zumindest in einem Lichtweg (der auch eine Streuung oder Reflexion umfassen kann) zwischen der Lichtquelle 120 und dem Lichtempfänger 124 liegen.

Fig. 1B zeigt eine schematische Darstellung eines erfindungsgemäßen Sensors zum Liefern einer Information über einen Vitalparameter eines Lebewesens gemäß einem zweiten Ausführungsbeispiel der vorliegenden Erfindung. Der Sensor gemäß der Fig. 1B ist in seiner Gesamtheit mit 150 bezeichnet. Da der Sensor 150 dem Sensor 100 gemäß Fig. 1A sehr ähnlich ist, sind gleiche Einrichtungen bzw. Signale bei den Sensoren 100, 150 mit gleichen Bezugszeichen bezeichnet und werden hier nicht noch einmal erläutert.

Der Sensor 150 umfasst, wie auch der Sensor 100, eine Lichtquelle 120, einen Lichtempfänger 124 sowie einen Beschleunigungssensor 130. Das von dem Beschleunigungssensor 130 gelieferte Beschleunigungssignal 136 wird hierbei verwendet, um das Lichtintensitätssignal 126 in Abhängigkeit von dem Beschleunigungssignal 136 zu erzeugen. Bei einem Ausführungsbeispiel des Sensors 150 wirkt das Beschleunigungssignal 136 beispielsweise auf die Lichtquelle 120, um die Lichtquelle 120 abzuschalten, falls das Beschleunigungssignal 136 anzeigt, dass eine auf den Sensor 150 wirkende Beschleunigung größer als eine maximal zulässige Beschleunigung ist. In diesem Fall wird kein Lichtintensitätssignal 126 erzeugt, oder das Lichtintensitätssignal 126 nimmt durch die Deaktivierung der Lichtquelle 120 einen minimalen Wert bzw. Dunkelwert an. Durch die genannte Maßnahme wird Energie, die zum Betrieb der Lichtquelle 120 benötigt wird, eingespart, wenn der Beschleunigungssensor 136 erkennt, dass die auf den Sensor 150 wirkende Beschleunigung zu groß ist, um ein zuverlässiges Lichtintensitätssignal 126 zu erzeugen. Alternativ oder zusätzlich kann das Beschleunigungssignal 136 ferner dem Lichtempfänger 124 zugeführt werden, um beispielsweise den Lichtempfänger 124 zu deaktivieren, wenn das Beschleunigungssignal 136 eine Beschleunigung anzeigt, die größer ist als eine maximal zulässige Beschleunigung. Somit wird beispielsweise durch eine direkte Einwirkung des Beschleunigungssensors 130 auf dem Lichtempfänger 124 verhindert, dass der Lichtempfänger 124 ein unzuverlässiges Lichtintensitätssignal 126 ausgibt, wenn die auf den Sensor wirkende Beschleunigung einen vorgegebenen Schwellenwert überschreitet.

Das Beschleunigungssignal 136 kann beispielsweise bewirken, dass der Lichtempfänger 124 kein Lichtintensitätssignal 126 mehr ausgibt, wenn die auf den Sensor 150 wirkende Beschleunigung zu groß (größer als eine maximal zulässige Beschleunigung) ist.

Alternativ dazu kann der Lichtempfänger ausgelegt sein, um einen vorher bestimmten Lichtintensitätswert weiterhin auszugeben, wenn das Beschleunigungssignal 136 eine unzulässig große Beschleunigung anzeigt. Ferner kann der Lichtempfänger 124 alternativ ausgelegt sein, um ein Fehlersignal auszugeben, wenn das Beschleunigungssignal 136 eine unzulässig große Beschleunigung anzeigt.

Durch die genannten Maßnahmen wird jeweils gewährleistet, dass das Lichtintensitätssignal 126 nicht unbemerkt ungültige Werte liefert, die in einer Verarbeitungseinrichtung, die das Lichtintensitätssignal 126 empfängt, zu einer Fehlinterpretation bzw. Falschmessung führen würden.

Fig. 2A zeigt eine schematische Darstellung einer erfindungsgemäßen Verarbeitungseinrichtung zum verknüpfenden Verarbeiten eines Lichtintensitätssignals und eines Beschleunigungssignals, gemäß einem dritten Ausführungsbeispiel der vorliegenden Erfindung. Die schematische Darstellung der Fig. 2A ist in ihrer Gesamtheit mit 200 bezeichnet. Eine Verarbeitungseinrichtung 210 ist ausgelegt, um von einem Lichtempfänger 124 ein Lichtintensitätssignal 126 zu empfangen. Die Verarbeitungseinrichtung 210 ist ferner ausgelegt, um von einem Beschleunigungssensor 130 ein Beschleunigungssignal 136 zu empfangen. Die Verarbeitungseinrichtung 210 ist ferner ausgelegt, um das Lichtintensitätssignal 126 und das Beschleunigungssignal 136 gemäß einem vorgegebenen Algorithmus zu verknüpfen, um als Ausgangssignal 220 entweder ein um beschleunigungsbedingte Effekte korrigierte Lichtintensitätssignal zu erhalten, oder um als Ausgangssignal eine um beschleunigungsbedingte Effekte korrigierte Information über den Vitalparameter zu erhalten.

So kann beispielsweise das Beschleunigungssignal verwendet werden, um Parameter einer Signalverarbeitungsanordnung, die das Lichtintensitätssignal 126 empfängt, um das Ausgangssignal 220 basierend darauf zu erzeugen, in Abhängigkeit von dem Beschleunigungssignal 136 einzustellen bzw. anzupassen. Ferner kann die Verarbeitungseinrichtung 210 ausgelegt sein, um das Lichtintensitätssignal 126 mit dem Beschleunigungssignal 136 beispielsweise additiv, subtraktiv oder multiplikativ zu verknüpfen. Ferner kann die Verarbeitungseinrichtung 210 zusätzlich oder alternativ ausgelegt sein, um beispielsweise das Beschleunigungssignal (beispielsweise über der Zeit) zu integrieren, um durch einmalige oder zweimalige Integration des Beschleunigungssignals eine Information über eine Geschwindigkeit oder einen Ort der Befestigungseinrichtung zu erhalten und um die genannte Information bei einer Bestimmung des Ausgangssignals zu berücksichtigen. Entsprechend kann das Lichtintensitätssignal 126 nicht nur mit dem Beschleunigungssignal 136 an sich, sondern auch mit einem durch einmalige oder mehrmalige Integration des Beschleunigungssignals 136 entstehende Signal kombiniert werden. Ferner kann bei der Berechnung des ersten Signals 220 alternativ oder zusätzlich zu dem Lichtintensitätssignal 126 an sich auch eine (kontinuierliche oder diskrete) zeitliche Ableitung des Lichtintensitätssignals 126 einfließen. Alternativ oder zusätzlich kann ferner das Lichtintensitätssignal 126 auch ein oder mehrmals (beispielsweise über der Zeit integriert werden, um das Ausgangssignal 220 zu erhalten.

Fig. 2B zeigt eine schematische Darstellung einer erfindungsgemäßen Verarbeitungseinrichtung zum verknüpfenden Verarbeiten eines Lichtintensitätssignals und eines Beschleunigungssignals, gemäß einem vierten Ausführungsbeispiel der vorliegenden Erfindung. Die schematische Darstellung gemäß der Fig. 2B ist in ihrer Gesamtheit mit 230 bezeichnet. Eine Verarbeitungseinrichtung 240 empfängt ein Lichtintensitätssignal 126 von einem Lichtempfänger 124 sowie ein Beschleunigungssignals 136 von einem Beschleunigungssensor 130. Die Verarbeitungseinrichtung 240 umfasst einen Referenzwertvergleicher 242, der das Beschleunigungssignal 136 mit einem maximal zulässigen Beschleunigungswert 244 vergleicht. In anderen Worten, die Vergleichseinrichtung 242 bestimmt, ob das Beschleunigungssignal 136 sich innerhalb eines zulässigen Bereichs befindet oder nicht.

Die Vergleichseinrichtung 242 liefert dann ein Vergleichsergebnis 246 an eine Ausgabeeinrichtung 248. Zeigt das Vergleichsergebnis 246 an, das die Beschleunigung innerhalb des zulässigen Bereichs liegt, so gibt die Ausgabeeinrichtung 248 das Lichtintensitätssignal 126 als Ausgangssignal 250 (beispielsweise unverändert) weiter. Zeigt das Vergleichsergebnis 246 hingegen an, dass die Beschleunigung außerhalb des zulässigen Bereichs liegt (beispielsweise also unzulässig groß ist) so gibt die Ausgabeeinrichtung 248 beispielsweise als Ausgangssignal 250 ein Fehlersignal aus. Alternativ dazu kann die Ausgabeeinrichtung 248 auch ausgelegt sein, um im Falle einer unzulässig großen Beschleunigung (also wenn das Vergleichsergebnis 246 eine unzulässig großen Beschleunigung anzeigt) weiterhin ein vorher ermitteltes Lichtintensitätssignal 126 ausgeben. In anderen Worten, die Ausgabeeinrichtung 248 kann ein Tor bzw. Datentor bzw. Latch umfassen das ein aktuelles Lichtintensitätssignal 126 weitergibt, so lange das Vergleichsergebnis 246 anzeigt, dass die Beschleunigung innerhalb des zulässigen Bereichs ist, und das eine Veränderung des Ausgangssignals 250 unterbindet, falls das Vergleichsergebnis 246 anzeigt, dass die Beschleunigung unzulässig groß ist.

Fig. 2C zeigt eine schematische Darstellung einer erfindungsgemäßen Verarbeitungseinrichtung zum verknüpfenden Verarbeiten eines Lichtintensitätssignals und eines Beschleunigungssignals gemäß einem fünften Ausführungsbeispiel der vorliegenden Erfindung. Die schematische Darstellung gemäß Fig. 2C ist in ihrer Gesamtheit mit 260 bezeichnet. Eine Verarbeitungseinrichtung 270 empfängt ein Lichtintensitätssignal 126 von einem Lichtempfänger 124 sowie ein Beschleunigungssignal 136 von einem Beschleunigungssensor 130. Die Verarbeitungseinrichtung 270 umfasst eine Zuverlässigkeitsbestimmungseinrichtung 272, die ausgelegt ist, um basierend auf dem Beschleunigungssignal 136 eine Zuverlässigkeitsinformation 274 zu erzeugen. Die Zuverlässigkeitsbestimmungseinrichtung 272 kann beispielsweise ausgelegt sein, um verschieden großen oder verschiedenartigen durch das Beschleunigungssignal 136 beschriebenen Beschleunigungen verschiedene Zuverlässigkeitsinformationen 274 zuzuordnen. Die Zuverlässigkeitsbestimmungseinrichtung 272 kann dabei ausgelegt sein, um neben einem Betrag der durch das Beschleunigungssignal 136 beschriebenen Beschleunigung auch eine Richtung der durch das Beschleunigungssignal 136 beschriebenen Beschleunigung mit zu berücksichtigen. In anderen Worten, das Beschleunigungssignal 136 kann eine Beschleunigung in mehreren Richtungen beschreiben, so dass die Zuverlässigkeitsbestimmungseinrichtung 274 gegebenenfalls (also optional) auch eine Richtung der Beschleunigung auswerten kann. Ferner kann die Zuverlässigkeitsbestimmungseinrichtung 272 optional bei einer Bestimmung der Zuverlässigkeitsinformation 274 auch das Lichtintensitätssignal 126 mit auswerten.

Die Zuverlässigkeitsbestimmungseinrichtung 272 kann beispielsweise ausgelegt sein, um bei Vorliegen einer großen Beschleunigung die Zuverlässigkeitsinformation 274 so einzustellen, dass diese eine geringe Zuverlässigkeit anzeigt, und um bei Vorliegen einer kleineren Beschleunigung die Zuverlässigkeitsinformation 274 so einzustellen, dass diese eine größere Zuverlässigkeit anzeigt. Ferner kann die Zuverlässigkeitsbestimmungseinrichtung 272 optional ausgelegt sein, um bei Vorliegen eines betragsmäßig großen Lichtintensitätssignals 126 die Zuverlässigkeitsinformation 274 so einzustellen, dass diese eine große Zuverlässigkeit anzeigt, und um bei Vorliegen eines kleineren Wertes des Lichtintensitätssignals 126 die Zuverlässigkeitsinformation 274 so einzustellen, dass diese eine geringere Zuverlässigkeit anzeigt.

Die Verarbeitungseinrichtung 270 umfasst ferner eine Ausgabeeinrichtung 278, die ausgelegt ist, um ein Ausgangssignal 280 zu erzeugen, das eine kombinierte Information trägt, die sowohl die Zuverlässigkeitsinformation 274 als auch das Lichtintensitätssignal 126 oder eine aus dem Lichtintensitätssignal 126 extrahierte Information umfasst. Beispielsweise kann die Ausgabeeinrichtung 278 ausgelegt sein, um als das Ausgangssignal 280 Datenpaare auszugeben, die eine aus dem Lichtintensitätssignal 126 abgeleitete Lichtintensität sowie eine zugeordnete Zuverlässigkeit, die aus der Zuverlässigkeitsinformation 274 hergeleitet ist, umfassen.

Alternativ kann die Verarbeitungseinrichtung 270 ferner zusätzlich eine Vitalparemter-Bestimmungseinrichtung 282 umfassen, die ausgelegt ist, um das Lichtintensitätssignal 126 zu empfangen, und um basierend auf dem Lichtintensitätssignal 126 eine Information über einen Vitalparameter an die Ausgabeeinrichtung 278 zu liefern. In diesem Fall ist die Ausgabeeinrichtung 278 bevorzugt ausgelegt, um als Ausgangssignal 278 einen Datenstrom zu liefern, der sowohl eine Information über den Vitalparameter als auch eine zugeordnete Zuverlässigkeitsinformation umfasst. In anderen Worten, in diesem Fall umfasst das Ausgangssignal bevorzugt Datenpaare, die eine Information über einen Vitalparameter sowie eine zugeordnete Information über die Zuverlässigkeit der Information über den Vitalparameter umfassen.

Bei einem weiteren bevorzugten Ausführungsbeispiel ist die die Verarbeitungseinrichtung 270 ausgelegt, um durch Verknüpfung des Lichtintensitätssignals 126 sowie des Beschleunigungssignals 136 zu ermitteln, ob ein Informationsinhalt des Lichtintensitätssignals 126 plausibel ist. Beispielsweise kann überprüft werden, ob Veränderungen in dem Lichtintensitätssignal 126 zeitlich mit dem Auftreten einer Beschleunigung korrelieren. In diesem Fall kann angenommen werden, dass die Veränderungen in dem Lichtintensitätssignal auf die Beschleunigung zurückzuführen sind, und somit für eine Auswertung nicht herangezogen werden sollten. Somit kann das Lichtintensitätssignal in diesem Fall als unzuverlässig gekennzeichnet werden. Tritt hingegen eine starke Beschleunigung auf, aber das Lichtintensitätssignal 226 zeigt zu dem Zeitpunkt, zu dem die Beschleunigung auftritt, keine signifikante Veränderung, so kann ferner davon ausgegangen werden, dass das Lichtintensitätssignal 126 trotz Vorliegen der vergleichsweise starken Beschleunigung (stärker als ein vorgegebener Beschleunigungsgrenzwert) zuverlässig ist. Somit kann insgesamt durch die Verarbeitungseinrichtung 270 überprüft werden, ob beispielsweise eine zeitliche Koordination zwischen Veränderungen in dem Lichtintensitätssignal 126 und dem Auftreten einer starken Beschleunigung (stärker als ein vorgegebener Beschleunigungs-Grenzwert) vorliegen. Nur falls ein zeitlicher Zusammenhang besteht, kann das Lichtintensitätssignal 126 als unzuverlässig gekennzeichnet werden, während in allen anderen Fällen das Lichtintensitätssignal 126 als zuverlässig gekennzeichnet wird.

Somit kann eine Plausibilitätsüberprüfung des Lichtintensitätssignals 126 erfolgen, und das Lichtintensitätssignal 126 wird beispielsweise dementsprechend als unzuverlässig gekennzeichnet oder nicht an eine weitere Verarbeitung weitergegeben, wenn signifikante Veränderungen (Veränderungen, die größer als ein vorgeschriebener Schwellenwert sind) in dem Lichtintensitätssignal 126 zeitlich kurz vor oder kurz nach dem Auftreten einer starken Beschleunigung (bzw. innerhalb eines vorgegebenen Zeitintervalls um das Auftreten einer starken Beschleunigung herum) auftreten.

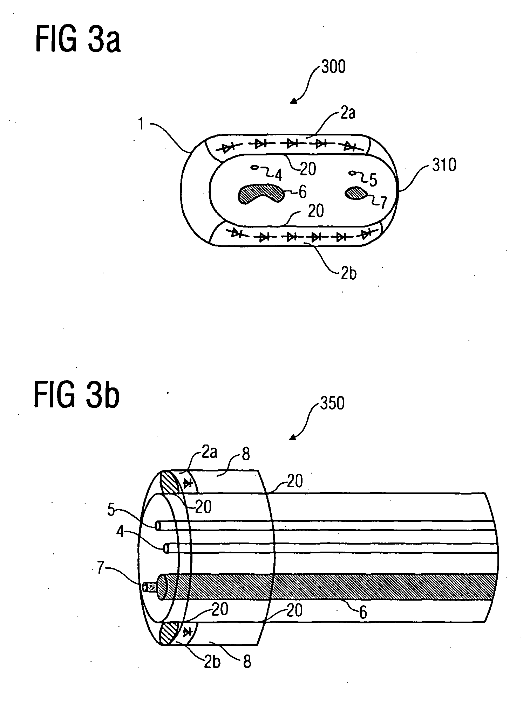

Zur weiteren Verdeutlichung wird im Folgenden anhand der Fig. 3A und 3B die räumliche Anordnung eines erfindungsgemäßen Sensors beschrieben, wenn dieser beispielsweise um einen menschlichen Arm (z. B. Unterarm) herum angebracht ist. Die Fig. 3A zeigt daher eine Querschnittsdarstellung eines erfindungsgemäßen Sensors, der um einen menschlichen Unterarm befestigt ist. Die Querschnittsdarstellung gemäß der Fig. 3A ist in ihrer Gesamtheit mit 300 bezeichnet. Eine Handgelenkmanschette 1, die bei dem Ausführungsbeispiel gemäß der Fig. 3A als Befestigungseinrichtung dient, umschließt zumindest teilweise einen menschlichen Arm 310. An der Handgelenkmanschette 1 ist eine Lichtquellenmatrix 2a angebracht, die als Lichtquelle dient. Die Lichtquellenmatrix 2a umfasst zumindest eine lichtemittierende Diode, bevorzugt aber eine Mehrzahl von lichtemittierenden Dioden, die ausgelegt sind, um Licht unterschiedlicher spektraler Zusammensetzung auszustrahlen. In anderen Worten, bei einem bevorzugten Ausführungsbeispiel ist eine erste lichtemittierende Diode der Lichtquellenmatrix 2a so ausgelegt, dass das von der ersten lichtemittierenden Diode erzeugte Licht ein Intensitätsmaximum einer ersten Lichtwellenlänge λ₁ aufweist. Eine zweite lichtemittierende Diode hingegen ist bevorzugt so ausgelegt, dass ein von der zweiten lichtemittierenden Diode ausgestrahltes Licht ein Intensitätsmaximum bei einer zweiten Lichtwellenlänge λ₂ aufweist, wobei sich die zweite Lichtwellenlänge λ₂ von der ersten Lichtwellenlänge λ₁ unterscheidet.
Die Handgelenksmanschette 1 umfasst ferner eine photoempfindliche Empfängermatrix 2b, die zumindest eine lichtempfindliche Diode umfasst. Bevorzugt umfasst die lichtempfindliche Empfängermatrix 2b allerdings eine Mehrzahl von lichtempfindlichen Dioden. Es wird ferner bevorzugt (ist aber nicht zwingend erforderlich), dass die lichtempfindlichen Dioden der Empfängermatrix 2b unterschiedliche spektrale Empfindlichkeiten aufweisen.

Ganz allgemein lässt sich hierbei festhalten, dass, es im Rahmen der vorliegenden Erfindung ausreichend ist, wenn die Lichtquellenmatrix 2a zumindest eine lichtemittierende Diode (oder eine andere Lichtquelle) umfasst, und wenn die photoempfindliche Empfängermatrix 2b zumindest eine lichtempfindliche Diode (oder ein anderes lichtempfindliches Element) umfasst. Es wird allerdings bevorzugt, dass die Lichtquellenmatrix 2a eine Mehrzahl von lichtemittierenden Dioden (oder anderen Lichtquellen) umfasst, und dass die photoempfindliche Empfängermatrix 2b eine Mehrzahl von lichtempfindlichen Dioden (oder anderen photoempfindlichen Elementen) umfasst. Weiterhin wird es bevorzugt (ist aber nicht zwingend erforderlicht) dass die Lichtquellenmatrix 2a Dioden (bzw. andere Lichtquellen) mit unterschiedlicher spektraler Verteilung des ausgesendeten Lichts umfasst. Ferner wird es bevorzugt (ist aber nicht zwingend erforderlich) dass die photoempfindliche Empfängermatrix 2b lichtempfindliche Dioden bzw. Photodioden (oder andere lichtempfindliche Elemente) mit unterschiedlicher spektraler Empfindlichkeit umfasst. Um einen spektralen Verlauf einer optischen Dämpfung zwischen der Lichtquellenmatrix 2a und der photoempfindliche Empfängermatrix 2b zu bestimmen, ist es im Übrigen ausreichend, wenn entweder die Lichtquellenmatrix 2a lichtemittierende Dioden mit unterschiedlicher spektraler Verteilung aufweist, oder wenn die photoempfindliche Empfängermatrix 2b lichtempfindliche Dioden mit unterschiedlicher spektraler Empfindlichkeit aufweist.

Ferner wird darauf hingewiesen, dass der Unterarm 310 einen Speichenknochen 6 (auch mit Radius bezeichnet) sowie einen Ellenknochen 7 (auch mit Ulna bezeichnet) umfasst. Ferner umfasst der Unterarm 310 eine Speichenschlagader 4 (auch mit Arteria radialis bezeichnet) sowie eine Ellenschlagader 5 (auch mit Arteria ulnaris bezeichnet). Die Speichenschlagader 4, die Ellenschlagader 5, die Speiche 6 und die Elle 7 sind in der aus der Medizin bekannten Weise in dem Unterarm 310 angeordnet.

Die Lichtquellenmatrix 2a (auch kurz als Lichtquelle bezeichnet) und die photoempfindliche Empfängermatrix 2b (auch kurz als Lichtempfänger bezeichnet) sind im Übrigen so an der Handgelenkmanschette 1 (auch als Befestigungseinrichtung bezeichnet) angeordnet, dass zumindest eine Arterie (also beispielsweise die Speichenschlagader 4 oder die Ellenschlagader 5) sich zwischen einer lichtemittierenden Diode (oder allgemein: einer Lichtquelle) der Lichtquellenmatrix 2a und einer lichtempfindlichen Diode (oder allgemein: einem lichtempfindlichen Element) der photoempfindliche Empfängermatrix 2b befindet, wenn die Handgelenkmanschette 1 an einem menschlichen Unterarm oder um ein menschliches Handgelenk befestigt ist.

An der Handgelenksmanschette 1 sind ferner zwei Elektroden bzw. Hautelektroden 20 angeordnet, so dass die Hautelektroden 20 in einer elektrisch leitfähigen Verbindung mit der Haut des Unterarms 310, oder des Handgelenks oder der Handwurzel stehen, wenn die Handgelenksmanschette 1 an dem Unterarm 310, an dem Handgelenk oder an der Handwurzel angebracht ist. Die Hautelektroden 20 sind ferner mit einer Einrichtung zur Impedanzmessung gekoppelt, um eine Impedanz zwischen den Hautelektroden 20 zu bestimmen, wie im Folgenden noch ausführlicher erläutert wird.

Fig. 3B zeigt ferner ein Schrägbild eines erfindungsgemäßen Sensors, der um einen menschlichen Unterarm befestigt ist. Die graphische Darstellung der Fig. 3B ist in ihrer Gesamtheit mit 350 bezeichnet. Da die graphische Darstellung 350 sich von der graphischen Darstellung 300 nur durch die gewählte Perspektive unterscheidet, tragen gleiche Einrichtungen bzw. Merkmale in den graphischen Darstellungen 300 bzw. 350 gleiche Bezugszeichen. Auf eine wiederholte Erläuterung wird daher hier verzichtet.

Es sei allerdings darauf hingewiesen, dass beispielsweise die Lichtquellenmatrix 2a derart an der Handgelenksmanschette 1 angebracht ist, dass die Lichtquellenmatrix 2a der Innenseite des Unterarms, der Innenseite des Handgelenks oder die Innenseite der Handwurzel benachbart ist, wenn die Handgelenkmanschette 1 an dem Unterarm 310, um das Handgelenk oder um die Handwurzel angebracht ist. Ferner wird es bevorzugt, dass die photoempfindliche Empfängermatrix 2b so an die Handgelenkmanschette 1 angeordnet ist, dass die lichtempfindliche Empfängermatrix 2b einer Außenseite des Unterarms, des Handgelenks oder der Handwurzel benachbart ist, wenn die Handgelenkmanschette 1 an dem Unterarm, um das Handgelenk oder um die Handwurzel angebracht ist.

Alternativ dazu kann die Lichtquellenmatrix 2a aber auch derart an der Handgelenkmanschette 1 angebracht sein, dass die Lichtquellenmatrix 2a der Außenseite des Unterarms 310, der Außenseite des Handgelenks oder der Außenseite der Handwurzel benachbart ist, wenn die Handgelenkmanschette 1 an dem Unterarm, um das Handgelenk oder um die Handwurzel angebracht ist. In diesem Fall ist die photoempfindliche Empfängermatrix 2b bevorzugt so an der Handgelenkmanschette 1 angeordnet, dass die photoempfindliche Empfängermatrix 2b der Innenseite des Unterarms, der Innenseite des Handgelenks oder der Innenseite der Handwurzel benachbart ist, wenn die Handgelenkmanschette 1 an dem Unterarm, um das Handgelenk oder um die Handwurzel angebracht ist.

Ferner wird es bevorzugt, dass die Handgelenkmanschette 1 so ausgelegt ist, dass die Handgelenkmanschette 1 um das Handgelenk fixiert ist, wenn die Handgelenkmanschette um das Handgelenk angebracht ist, dass die Handgelenkmanschette 1 also nicht in Richtung der Handwurzel oder in Richtung des Unterarms verschiebbar ist, wenn die Handgelenkmanschette um das Handgelenk herum angebracht ist. Dadurch ist sichergestellt, dass die Messung stets an dem optimalen Ort, nämlich in direkter Nähe des Handgelenks, erfolgt.

Aus der Fig. 3B ist ersichtlich, dass an der Handgelenkmanschette 1 ferner ein Beschleunigungssensor 8 angebracht ist. Dabei können verschiedene Orte für den Beschleunigungssensor gewählt werden. Bei einem bevorzugen Ausführungsbeispiel ist der Beschleunigungssensor 8 benachbart zu der Lichtquellenmatrix 2a angeordnet, so dass der Beschleunigungssensor 8 und die Lichtquellenmatrix 2a sich auf der gleichen Seite (Innenseite oder Außenseite) des Unterarms, des Handgelenks oder der Handwurzel befinden. Dadurch ist beispielsweise sichergestellt, dass der Beschleunigungssensor eine Beschleunigung, die auf die Lichtquellenmatrix 2a einwirkt, aufnimmt. Es wurde nämlich erkannt, dass eine Verschiebung der Lichtquellenmatrix 2a gegenüber dem Unterarm, dem Handgelenk oder der Handwurzel einen besonders starken Einfluss auf das durch die photoempfindliche Empfängermatrix 2b gelieferte Lichtintensitätssignal aufweist.

Bei einem weiteren bevorzugten Ausführungsbeispiel ist der Beschleunigungssensor 8 benachbart zu der photoempfindliche Empfängermatrix 2b angeordnet, so dass sich der Beschleunigungssensor 8 auf der gleichen Seite (Innenseite oder Außenseite) des Unterarms, des Handgelenks oder der Handwurzel befindet wie die photoempfindliche Empfängermatrix. Auch eine solche Anordnung ist besonders vorteilhaft, da ein großer Fehler in dem Lichtintensitätssignal entstehen kann, wenn sich die photoempfindliche Empfängermatrix 2b gegenüber dem Unterarm, dem Handgelenk oder der Handwurzel durch eine Beschleunigung verschiebt.

Bei einem weiteren bevorzugten Ausführungsbeispiel können auch zwei oder mehrere Beschleunigungssensoren an verschiedenen Stellen der Handgelenkmanschette 1 angeordnet sein, beispielsweise sowohl benachbart zu der Lichtquellenmatrix 2a als auch benachbart zu der photoempfindlichen Empfängermatrix 2b.

Die Signale der zwei oder mehreren Beschleunigungssensoren können dann kombiniert werden, oder können verwendet werden, um Beschleunigungen in verschiedene Richtungen zu beschreiben bzw. zu erfassen.

In anderen Worten, die vorliegende Erfindung schafft gemäß einem Ausführungsbeispiel einen am Handgelenk tragbaren Photoplethysmographen basierend auf dem Transmissionsprinzip. Der Plethysmograph umfasst bei einem Beispiel eine aus mehreren Blöcken bestehende matrixförmige Anordnung mehrerer Lichtquellen unterschiedlicher Wellenlänge, die beispielsweise durch die Lichtquellenmatrix 2a gebildet wird. Der Plethysmograph umfasst ferner gemäß einem Ausführungsbeispiel eine aus mehreren Blöcken bestehende, matrixförmige Anordnung photoempfindlicher Elemente, deren Spektrum (bzw. spektrale Empfindlichkeit) auf die (z. B. bei den Lichtquellen) verwendeten Wellenlängen abgestimmt ist. Die matrixförmige Anordnung photoempfindlicher Elemente wird bei einem Ausführungsbeispiel durch die photoempfindliche Empfängermatrix 2b gebildet.

Der Photoplethysmograph umfasst ferner bei einem Ausführungsbeispiel einen Beschleunigungssensor für jede der drei Achsen (bzw. Richtungen) im Raum. Alternativ kann der Photoplethysmograph aber auch nur einen oder zwei Beschleunigungssensoren für eine Richtung oder für zwei Richtungen umfassen. Die Beschleunigungssensoren (bzw. der Beschleunigungssensor) dienen der Verbesserung der Signalqualität und liefern einen Maßstab zur Bewertung einer Plausibilität eines aufgenommenen Plethysmogramm.

Die Verwendung von Lichtquellen verschiedener Wellenlängen ermöglicht gemäß einem Ausführungsbeispiel eine Anpassung des Photoplethysmographen an eine Hautfarbe sowie an eine Anatomie eines Handgelenks und lässt ferner Rückschlüsse auf im Blutbestandteile (beispielsweise von Blut in einer Arterie zwischen der Lichtquellenmatrix 2a und der photoempfindlichen Empfängermatrix 2b) zu.

Gemäß einem weiteren bevorzugten Ausführungsbeispiel sind die auf der Hautoberfläche (beispielsweise des Unterarms, des Handgelenks oder der Handwurzel) aufliegenden und die Lichtquellen bzw. die photoempfindlichen Elemente beinhaltenden Gehäuse (bzw. ist das die Lichtquellen und die photoempfindlichen Elemente beinhaltende Gehäuse) derart gestaltet, dass die Gehäuse (bzw. das Gehäuse) als mindestens zwei Hautelektroden 20 zur Messung einer Hautimpedanz verwendet werden können (bzw. kann).

Von der Hautimpedanz wird gemäß einem Ausführungsbeispiel der vorliegenden Erfindung auf einen Wasseranteil in einem Gewebe geschlossen.

Aus dem Wasseranteil in dem Gewebe kann dann optional beispielsweise auf einen Grad einer Austrocknung (Exsikkation) eines Patienten, sowie auf eine Blutviskosität und auf damit verbundene Schlaganfallrisiken geschlossen werden.

Die vorliegende Erfindung basiert somit auf dem Konzept, am Handgelenk (z.B. eines Menschen oder eines Lebewesens) ein Plethysmogramm zu detektieren, indem eine Lichtquelle 120, 2a geeigneter Wellenlängen mit geeigneter Ansteuerung an einer Außenseite des Handgelenks derart gegenüber einem photoempfindlichen Element 124, 2b auf der Innenseite des Handgelenks angeordnet ist, dass sich mindestens eine der Armarterien (Arteria radialis 4 oder Arteria ulnaris 5) zwischen der Lichtquelle 120, 2a und dem photoempfindlichen Element 124, 2b befindet.

Die Erfindung basiert gemäß einem weiteren Aspekt auf dem Konzept, dass über eine Messung der Hautimpedanz auf den Wasseranteil in dem Gewebe und daraus auf den Grad der Austrocknung (Exsikkation) und auf die Blutviskosität sowie auf ein damit verbundenes Schlaganfallrisiko geschlossen werden kann.

Ein weiterer Kerngedanke der vorliegenden Erfindung besteht darin, dass mit einem geeigneten Ansteuerverfahren der Lichtquelle 120 bzw. der Lichtquellenmatrix 2a sowie des Lichtempfängers 124 bzw. der photoempfindlichen Empfängermatrix 2b unter Verwendung verschiedener Wellenlängen auf die Blutbestandteile geschlossen werden kann.

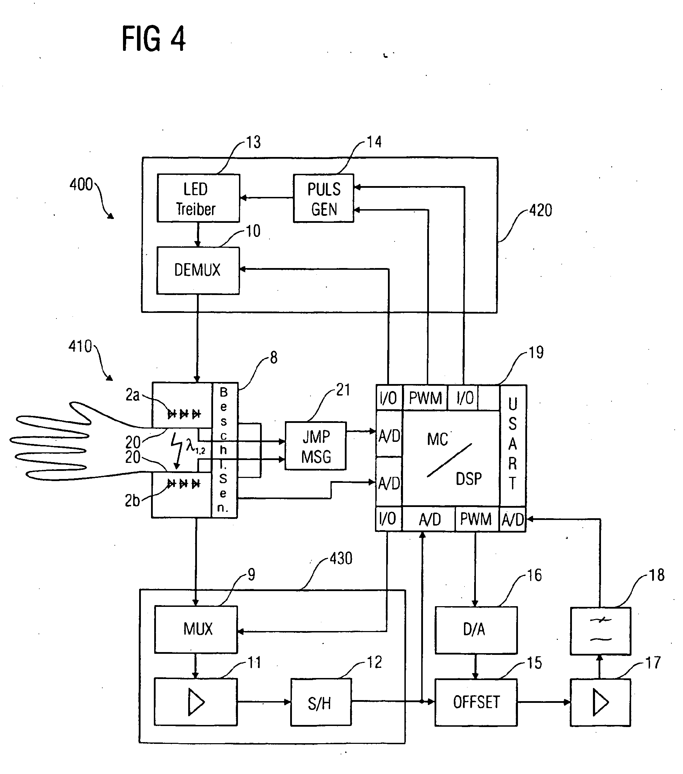

Fig. 4 zeigt eine schematische Darstellung eines erfindungsgemäßen Sensors einschließlich einer Schaltungsanordnung zur Ansteuerung der Lichtquelle und zur Auswertung des Lichtintensitätssignals. Die Anordnung gemäß der Fig. 4 ist in ihrer Gesamtheit mit 400 bezeichnet.

Kern der Anordnung 400 ist ein Messaufnehmer 410, der beispielsweise eine Handgelenkmanschette 1, eine Lichtquellenmatrix 2a, eine photoempfindliche Empfängermatrix 2b, einen Beschleunigungssensor 8 sowie optional zumindest zwei Hautelektroden 20 umfasst, wie dies beispielsweise anhand der Fig. 1A, 1B, 3A und 3B beschrieben wurde.

Bei einem bevorzugten Ausführungsbeispiel (aber nicht notwendigerweise) sind die Lichtquellenmatrix 2a sowie die photoempfindliche Empfängermatrix 2b ausgelegt, um zumindest zwei Lichtwellenlängen λ₁, λ₂ zu verwenden. Alternativ kann aber bei einem einfachen Ausführungsbeispiel auch nur eine Lichtwellenlänge λ verwendet werden.

Die Schaltungsanordnung 400 wird durch eine Mikrocontroller und/oder einen digitalen Signalprozessor 19 gesteuert, der beispielsweise eine Mehrzahl von digitalen Ausgangsleitungen bereitstellt, und der ferner für sich allein oder in Kombination mit weiteren Peripherieelementen ausgelegt ist, um mehrere analoge Signale einzulesen. Die Steuerung der Schaltungsanordnung 400 kann alternativ durch eine diskrete analoge und/oder digitale Schaltung erfolgen.

Die Schaltungsanordnung 400 umfasst ferner eine Ansteuereinheit 420, die ausgelegt ist, um die einen oder mehreren lichtemittierten Dioden der Lichtquellenmatrix 2a anzusteuern. Die Ansteuerschaltung 420 umfasst einen Pulsgenerator 14, der ausgelegt ist, um Impulse zur Ansteuerung eines LED-Treibers 13 zu erzeugen. Der LED-Treiber 13 stellt in Verbindung mit dem Pulsgenerator 14 Spannungsimpulse oder Stromimpulse zur Verfügung, die der Ansteuerung der lichtemittierenden Diode der Lichtquellenmatrix 2a dienen. Umfasst die Lichtquellenmatrix 2a mehr als eine Diode, so verteilt ein Demultiplexer 2a die durch den LED-Treiber 13 erzeugten Spannungsimpulse oder Stromimpulse auf die lichtemittierenden Dioden der Lichtquellenmatrix 2a. Beispielsweise kann der Demultiplexer ausgelegt sein, um einen von dem LED-Treiber 13 gelieferten Spannungsimpuls oder Stromimpuls an genau eine ausgewählte lichtemittierende Diode aus einer Mehrzahl von lichtemittierenden Dioden oder an genau eine ausgewählte Gruppe von lichtemittierenden Dioden aus einer Mehrzahl von Gruppen von lichtemittierenden Dioden der Lichtquellenmatrix 2a weiterzuleiten. Der Demultiplexer 10 empfängt im Übringen eine Auswahlinformation von dem Mikrocontroller oder digitalen Signalprozessor 19, die festlegt, welche lichtemittierende Diode oder Gruppe von lichtemittierenden Dioden mit einem Spannungsimpuls oder Stromimpuls angeregt werden soll. Der Pulsgenerator 14 wird ferner bei einem bevorzugten Ausführungsbeispiel durch den Mikrocontroller oder den digitalen Signalprozessor 19 angesteuert, wodurch beispielsweise eine Impulsdauer und/oder eine Impulsintensität der an die lichtemittierenden Dioden geleitenden Spannungspulse oder Strompulse festgelegt wird.

Die Schaltungsanordnung 400 umfasst ferner eine Empfangseinrichtung 430, die mit der photoempfindlichen Empfängermatrix 2b gekoppelt ist, und die ausgelegt ist, um die von der photoempfindlichen Empfängermatrix gelieferten Spannungssignale oder Stromsignale auszuwerten. Bei einem bevorzugten Ausführungsbeispiel umfasst die Empfängerschaltung 430 einen Multiplexer 9, der ausgelegt ist, um ein Signal von einer lichtempfindlichen Diode der photoempfindlichen Empfängermatrix 2b (oder von einer Gruppe von lichtempfindlichen Dioden der photoempfindlichen Empfängermatrix 2b) zur Weiterleitung an einen Verstärker 11 auszuwählen. Der Multiplexer 9 wird dabei bevorzugt durch den Mikrocontroller oder digitalen Signalprozessor 19 angesteuert. Die Empfängerschaltung 430 umfasst ferner eine Abtast- und Halteschaltung (Sample-and-Hold-Schaltung) 12, die mit dem Ausgang des Verstärkers 11 gekoppelt ist, und die somit ausgelegt ist, um ein von einer durch den Multiplexer 9 ausgewählten lichtempfindlichen Diode geliefertes und durch den Verstärker 11 verstärktes Signal abzutasten und zu halten. Der Ausgang der Abtast- und Halteschaltung 12 ist ferner mit einem analogen Eingang des Mikrocontrollers oder digitalen Signalprozessors 19 gekoppelt, wobei das von der Abtast- und Halteschaltung 12 gelieferte Signal bevorzugt in ein digitales Signal umgesetzt wird.

Alternativ dazu kann selbstverständlich auch ein externer Analog-Digital-Wandler eingesetzt werden, der mit dem Mikrocontroller oder digitalen Signalprozessor 19 gekoppelt ist.

Das Ausgangssignal der Abtast- und Halteschaltung 12 wird ferner bei einem bevorzugten Ausführungsbeispiel einer Offset-Schaltung 15 zugeführt. Die Offset-Schaltung 15 ist ausgelegt, um das Ausgangssignal der Abtast- und Halteschaltung 12 um einen Offset zu verschieben, also um beispielsweise einen Gleichanteil in dem Offset-Signal zu verringern oder zu entfernen. Die Offsetschaltung 15 empfängt beispielsweise ein Signal von einem Digital-Analog-Wandler 16, der durch den Mikrocontroller oder digitalen Signalprozessor 19 angesteuert wird. Beispielsweise umfasst der Mikrocontroller oder digitale Signalprozessor 19 eine Einrichtung zur Pulsbreitenmodulation (PWM), um die Bereitstellung des Signals für die Offsetschaltung 15 zu ermöglichen. In diesem Fall kann der Digital-Analog-Wandler 16 beispielsweise lediglich ein Tiefpassfilter umfassen, um das von der Pulsbreitenmodulationsschaltung gelieferte pulsbreitenmodulierte Signal in eine entsprechende Gleichspannung umzusetzen. Alternativ dazu kann allerdings auch ein herkömmlicher Analog-Digital-Wandler eingesetzt werden, der beispielsweise von dem Mikrocontroller oder digitalen Signalprozessor ein digitales Signal empfängt, und der basierend darauf ein Eingangssignal für die Offsetschaltung 15 zur Verfügung stellt. In anderen Worten, die Offset-schaltung 15 bildet beispielsweise eine Differenz zwischen dem Ausgangssignal der Abtast- und Halteschaltung 12 und dem von der Digital-Analog-Wandlerschaltung 16 gelieferten Signal. Das Ergebnis der Differenzbildung, also das Ausgangssignal der Offsetschaltung 15, wird einer Serienschaltung aus einem Verstärker 17 und einem Tiefpassfilter 18 zugeführt. Durch die genannte Schaltungsanordnung wird erreicht, dass der Verstärker lediglich einen Wechselanteil des Ausgangssignals der Abtast- und Halteschaltung 12 empfängt, und dass somit der Wechselanteil des Ausgangssignals der Abtast- und Halteschaltung verstärkt und gefiltert wird. Das von dem Filter 18 gelieferte Ausgangssignal wird einer Analog-Digital-Wandlung zugeführt, wobei der Mikrocontroller oder digitale Signalprozessor 19 einen Analog-Digital-Wandler umfassen kann oder mit einem derartigen Analog-Digital-Wandler gekoppelt sein kann, um das Ausgangssignal des Filters 18 in ein digitales Signal zu wandeln.

Die Schaltungsanordnung 400 ist somit ausgelegt, um eine optische Dämpfung zwischen der Lichtquellenmatrix 2a und der photoempfindlichen Empfängermatrix 2b für zumindest eine Lichtwellenlänge und für zumindest ein Paar von Lichtquellen (z. B. zumindest einer lichtemittierenden Diode) und Lichtempfängern (z. B. zumindest einer lichtempfindlichen Diode) zu bestimmen. Durch die Verwendung einer Demultiplexerschaltung 10 und einer Multiplexerschaltung 9 kann im Übrigen unter Verwendung einer einfachen Hardware die Dämpfung zwischen der Lichtquellenmatrix 2a und der photoempfindlichen Empfängermatrix 2b für eine Mehrzahl von Lichtwellenlängen λ bzw. für eine Mehrzahl von geometrischen Ausbreitungswegen ermittelt werden.

Die Schaltungsanordnung 400 umfasst ferner einen Beschleunigungssensor 8, der mit der Lichtquellenmatrix 2a und/oder der photoempfindlichen Empfängermatrix 2b mechanisch gekoppelt ist. Der Beschleunigungssensor liefert damit eine Information, die die auf die Lichtquellenmatrix 2a oder auf die photoempfindliche Empfängermatrix 2b wirkende Beschleunigung beschreibt. Der Mikrocontroller oder digitale Signalprozessor 19 empfängt die Information über die Beschleunigung typischerweise als ein analoges Signal, und ist ausgelegt, um die Information über die Beschleunigung in ein digitales Signal zu wandeln und bei der Auswertung der von der photoempfindlichen Empfängermatrix 2b gelieferten Information mit zu berücksichtigen, wie dies bereits oben ausführlich erläutert wurde.

Der Mikrocontroller oder digitale Signalprozessor 19 umfasst ferner einen universellen seriellen, synchronen oder asynchronen Sender und/oder Empfänger (USART) zur Kommunikation mit weiteren Komponenten eines Systems. Im Übrigen sei darauf hingewiesen, dass der Mikrocontroller oder digitale Signalprozessor 19 typischerweise ausgelegt bzw. programmiert ist, um basierend auf der von der photoempfindlichen Empfängermatrix 2b gelieferten Information eine Information über einen Vitalparameter des Menschen, der den erfindungsgemäßen Sensor trägt, bereitzustellen. Alternativ dazu kann der Mikrocontroller oder digitale Signalprozessor 19 auch eine Zwischeninformation bestimmen bzw. bereitstellen, aus der der Vitalparameter bzw. die Information über den Vitalparameter ableitbar ist.

Der Sensor 410 umfasst ferner (optional) zwei Hautelektroden 20, die an dem Sensor 410 angeordnet sind, um in Kontakt mit einer Haut eines Lebewesens, das den Sensor 410 trägt, zu stehen. Eine Schaltungsanordnung 21 ist mit den Hautelektroden 20 gekoppelt, um eine Impedanzmessung einer Impedanz zwischen den Hautelektroden 20 durchzuführen. Die Schaltungsanordnung 21 zur Impedanzmessung liefert im Übrigen eine Information über die Impedanz an den Mikrocontroller oder digitalen Signalprozessor 19. Bevorzugt liefert die Schaltungsanordnung 21 zur Impedanzmessung ein analoges Signal, das einem Analogeingang des Mikrocontrollers oder digitalen Signalprozessors 19 zugeführt wird.

Die Auswertung bzw. Verwendung der genannten Information über die Impedanz zwischen den Hautelektroden 20 wird im Folgenden noch beschrieben.

Zusammenfassend lässt sich also festhalten, dass ein geeigneter Mikrocontroller, beispielsweise ein digitaler Signalprozessor 19, die Ansteuerung der einzelnen Komponenten der Anordnung 400 sowie die Aufzeichnung, Verarbeitung und Auswertung der sich aus der Anordnung 400 ergebenen Signalverläufe übernimmt.

Die Schaltungsanordnung 400 umfasst somit einen Pulsgenerator 14, der geeignete Spannungsverläufe zur Ansteuerung des LED-Treibers 13 erzeugt. Der Demultiplexer 10 übernimmt die Verteilung der erzeugten Signale auf die einzelnen in einer Matrix angeordneten und auf Blöcke aufgeteilten Lichtquellen 2a. Die Signale der Beschleunigungssensoren 8 (bzw. eines Beschleunigungssensors 8) werden von dem Mikrocontroller oder digitalen Signalprozessor 19 digitalisiert und verarbeitet. Eine Schaltung 21 erhält die Signale der Hautelektroden 20 und führt diese in geeigneter Art und Weise dem Mikrocontroller oder digitalen Signalprozessor zu, der die Signale der Hautelektroden digitalisiert und verarbeitet. Der Multiplexer 9 sorgt für die Aufnahme und Weiterleitung der Signale von den einzelnen in einer Matrix angeordneten und auf Blöcke aufgeteilten lichtempfindlichen Elemente 2b an das Abtasthalteglied 12. Nach dem Abtasthalteglied 12 wird das Signal von dem Mikrocontroller oder digitalen Signalprozessor 19 digitalisiert und verarbeitet. Das Signal des Abtasthalteglieds 12 wird auf eine Offset-Schaltung 15 geführt, welche über den Digital-Analog-Umsetzer 16 von dem Mikrocontroller oder digitalen Signalprozessor 19 angesteuert wird. Anschließend wird das Signal mit einer Schaltung 17 verstärkt, und mit einer Schaltung 18 gefiltert. Danach wird das Signal von dem Mikrocontroller oder digitalen Signalprozessor 19 digitalisiert und verarbeitet.

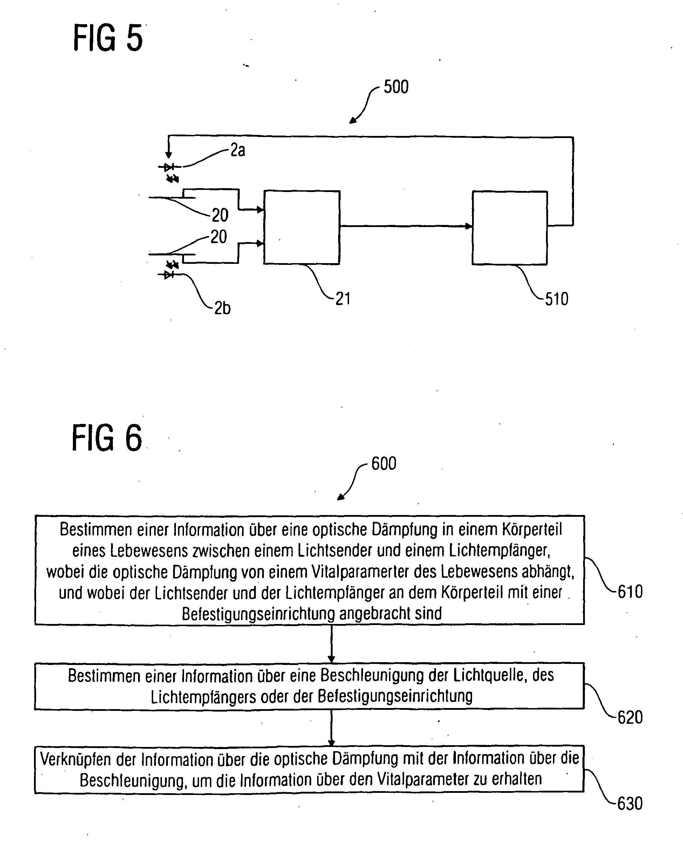

Fig. 5 zeigt ein Blockschaltbild einer erfindungsgemäßen Anordnung zur Einstellung einer von einer Lichtquelle abgegebenen Lichtmenge basierend auf einer Messung der Hautimpedanz.

Die Schaltungsanordnung gemäß der Fig. 5 ist in ihrer Gesamtheit mit 500 bezeichnet. Die Schaltungsanordnung 500 ist zur Verwendung in einem erfindungsgemäßen Sensor zu Bestimmung eines Vitalparameters des Lebewesens geeignet. Entscheidend für die Einsetzbarkeit der Schaltungsanordnung 500 ist die Tatsache, das ein Sensor zur Bestimmung eines Vitalparameters eine Lichtquelle 2a (z.B. in Form einer einzelnen Lichtquelle bzw. lichtemittierten Diode, oder in Form einer Lichtquellenmatrix) sowie einen Lichtempfänger 2b (z. B. in Form eines einzelnen Lichtempfängers bzw. einer einzelnen lichtempfindlichen Diode, oder in Form einer photoempfindlichen Empfängermatrix) umfasst, wobei Gewebe eines Körperteils von dem von der Lichtquelle 2a ausgestrahlten Licht durchstrahlt wird, um von dem Lichtempfänger 2b empfangen bzw. detektiert zu werden. Ferner sind typischerweise die Lichtquelle 2a und der Lichtempfänger 2b an einer Befestigungseinrichtung angebracht, die ihrerseits zur Anbringung an dem Körperteil ausgelegt ist. Die Befestigungseinrichtung, die die Lichtquelle 2a oder den Lichtempfänger 2b trägt, umfasst ferner zwei Hautelektroden 20, die mit der Befestigungseinrichtung verbunden sind, um in einem elektrisch leitfähigen Kontakt mit einer Hautoberfläche des von der Befestigungseinrichtung umschlossenen Körperteils zu stehen, wenn die Befestigungseinrichtung an dem Körperteil angebracht ist.

Die Hautelektroden 20 sind ferner bevorzugt in das Gehäuse der Befestigungseinrichtung, die die Lichtquelle 2a bzw. den Lichtempfänger 2b trägt und/oder häust, integriert. In anderen Worten, die Hautelektroden 20 bilden beispielsweise einen Teil einer Oberfläche des Gehäuses der Befestigungseinrichtung.

Eine Impedanzmessschaltung 21 ist elektrisch (bevorzugt elektrisch leitfähig) mit den Hautelektroden 20 gekoppelt, und ist ausgelegt, um eine Impedanz zwischen den Hautelektroden 20 zu bestimmen. Die Impedanzbestimmungsschaltung 21 kann beispielsweise ausgelegt sein, um nur einen Realteil einer Impedanz zwischen den Hautelektroden 20 zu bestimmen, um nur einen Imaginärteil einer Impedanz zwischen den Hautelektroden 20 zu bestimmen, oder um sowohl einen Realteil als auch einen Imaginärteil einer Impedanz zwischen den Hautelektroden 20 zu bestimmen. Es hat sich nämlich gezeigt, dass die Impedanz zwischen den Hautelektroden 20 beispielsweise ein Maß für einen Wasseranteil in einem Gewebe, das sich zwischen den Hautelektroden 20 befindet, darstellt, und dass ferner das Maß für den Wassergehalt in dem Gewebe eine optische Dämpfung beim Durchgang von Licht von der Lichtquelle 2a zu dem Lichtempfänger 2b beschreibt.

In anderen Worten, die Impedanzbestimmungseinrichtung 21 ermöglicht zusammen mit den Hautelektroden 20 ganz allgemein eine Bestimmung (bzw. Abschätzung) einer optischen Dämpfung zwischen der Lichtquelle 2a und dem Lichtempfänger 2b, wobei die Bestimmung der optischen Dämpfung auf nichtoptischen Wege erfolgt. In anderen Worten, die Bestimmung der Dämpfung erfolgt bevorzugt durch Messung einer elektrischen Eigenschaft des Körperteils. Alternativ dazu ist auch eine optische Messung der optischen Dämpfung möglich.
Die Schaltungsanordnung 500 umfasst ferner eine Lichtmengeneinstelleinrichtung 510, die mit der Impedanzmesseinrichtung 21 gekoppelt ist, um von der Impedanzmesseinrichtung 21 eine Information über eine Hautimpedanz zwischen den Elektroden 20 zu empfangen. Die LichtmengeneinstellEinrichtung 510 ist ferner ausgelegt, um auf die Ansteuerung der Lichtquelle 2a einzuwirken (bzw. die Lichtquelle 2a anzusteuern), um die von der Lichtquelle 2a abgestrahlte Lichtenergie oder Lichtleistung in Abhängigkeit von der von der Impedanzbestimmungseinrichtung 21 gelieferten Information einzustellen. Bei einem bevorzugten Ausführungsbeispiel ist die Lichtmengeneinstelleinrichtung 510 ausgelegt, um die von der Lichtquelle 2a in das Körperteil eingestrahlte Lichtenergie bzw. Lichtleistung auf einen großen Wert einzustellen, wenn die Impedanz zwischen den Hautelektroden 20 einen geringen Wert aufweist, und um die in das Körperteil eingestrahlte Lichtenergie oder Lichtleistung auf einen vergleichsweise dazu geringeren Werte einzustellen, wenn die Impedanz zwischen den Hautelektroden 20 einen vergleichsweise größeren Wert annimmt. Bei einem alternativen Ausführungsbeispiel ist die Lichtmengeneinstell-Einrichtung 510 ausgelegt, um die von der Lichtquelle 2a in das Körperteil eingestrahlte Lichtleistung derart einzustrahlen, dass die Lichtleistung bei Vorliegen einer größeren Impedanz zwischen den beiden Hautelektroden 20 einen größeren Wert annimmt als bei Vorliegen einer vergleichsweise kleineren Impedanz zwischen den Hautelektroden 20.

Bei einem weiteren bevorzugten Ausführungsbeispiel ist die Lichtmengeneinstell-Einrichtung ausgelegt, um basierend auf der von der Impedanzbestimmungseinrichtung 21 gelieferten Information über die Impedanz zwischen den Elektroden 20 eine Information über einen Wasseranteil in dem Gewebe des Körperteils zwischen den Hautelektroden 20 abzuleiten, und um basierend auf der Information über den Wasseranteil in dem Gewebe die von der Lichtquelle 2a in den Körperteil eingestrahlte Lichtenergie oder Lichtleistung einzustellen.

Bei einem weiteren bevorzugten Ausführungsbeispiel ist die Lichtmengeneinstell- Einrichtung 510 ausgelegt, um basierend auf der Information über den Wasseranteil in dem Gewebe des Körperteils zwischen den Hautelektroden 20 eine Information über eine optische Dämpfung in dem Gewebe des Körperteils zwischen den Hautelektroden 20 zu bestimmen, und um die von der Lichtquelle 2a in das Körperteil eingestrahlte Lichtenergie oder Lichtleistung in Abhängigkeit von der Information über die optische Dämpfung in dem Gewebe des Körperteils abzuleiten.

In anderen Worten, die durch die Lichtquelle 2a in den Körperteil eingestrahlte Lichtenergie oder Lichtleistung kann in einem mehrstufigen Prozess bestimmt werden, bei dem zwei oder mehr Schritte zu einem einzigen Schritt zusammengefasst werden können. Die möglichen einzelnen Schritte umfassen: Bestimmen der Impedanz zwischen zwei Hautelektroden, die mit dem Körperteil in elektrischem (bzw. elektrisch leitfähigem) Kontakt stehen; Bestimmen eines Wasseranteils in dem Gewebe des Körperteils basierend auf der Information über die Impedanz zwischen den Hautelektroden; Bestimmen einer Information über eine optische Dämpfung in dem Körperteil basierend auf der Information über den Wasseranteil in dem Gewebe des Körperteils; und Einstellen der Lichtenergie oder Lichtleistung basierend auf der Informationen über die optische Dämpfung in dem Körperteil.

Eine Bestimmung der Information über den Wasseranteil in dem Körperteil sowie eine Bestimmung der Information über die optische Dämpfung in dem Körperteil können optional entfallen, d. h. es kann eine Einstellung der Lichtenergie oder Lichtleistung der Lichtquelle 2a direkt basierend auf der Impedanz zwischen den Hautelektroden erfolgen. In anderen Worten, ein Realteil der Impedanz, ein Imaginärteil der Impedanz, ein Betrag der Impedanz oder eine Phase der Impedanz können beispielsweise durch die Lichtmengeneinstellungseinrichtung 510 auf eine Lichtenergie oder Lichtleistung der Lichtquelle 2a abgebildet werden. Die Abbildung kann beispielsweise durch einen funktionale Zusammenhang oder unter Verwendung einer Wertetabelle erfolgen, wobei jeweils einer bestimmten Impedanz (bzw. einem Realteil, einem Imaginärteil, einem Betrag oder einer Phase der Impedanz) eine Lichtenergie oder eine Lichtleistung zugeordnet wird.

Fig. 6 zeigt ein Flussdiagramm eines erfindungsgemäßen Verfahrens zum Liefern einer Information über einen Vitalparameter eines Lebewesens.

Das Verfahren gemäß der Fig. 6 ist seiner Gesamtheit mit 600 bezeichnet. Das Verfahren 600 umfasst ein Bestimmen 610 einer Information über eine optische Dämpfung in einem Körperteil des Lebewesens zwischen einem Lichtsender und einem Lichtempfänger, wobei die optische Dämpfung von einem Vitalparameter des Lebewesens abhängt. Der Lichtsender und der Lichtempfänger sind an dem Körperteil mit einer Befestigungseinrichtung angebracht.

Das Verfahren umfasst ferner in einem zweiten Schritt 620 ein Bestimmen einer Information über eine Beschleunigung der Lichtquelle, des Lichtempfängers oder der Befestigungseinrichtung.

Das Verfahren 600 umfasst ferner in einem dritten Schritt 630 ein Verknüpfen der Information über die optischen Dämpfung mit der Information über die Beschleunigung, um eine Information über den Vitalparameter zu erhalten.

Es sei ferner darauf hingewiesen, dass das Verfahren 600 um all diejenigen Schritte ergänzt werden kann, die von der oben beschriebenen erfindungsgemäßen Vorrichtung durchgeführt werden.

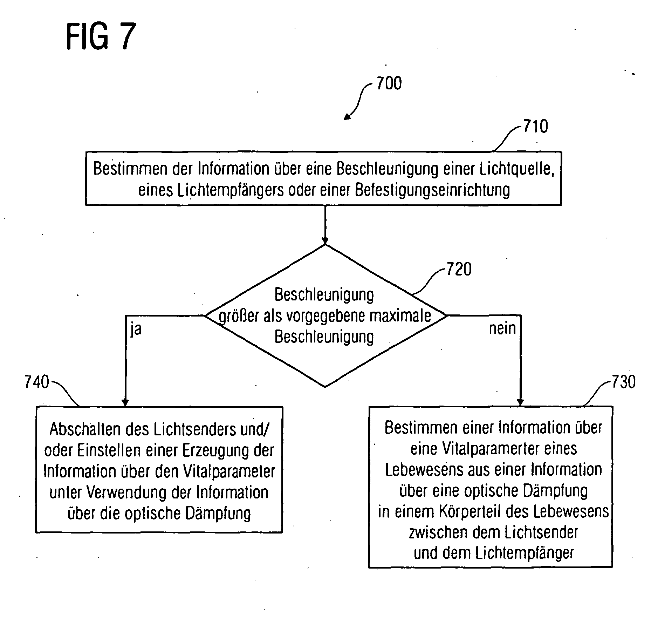

Fig. 7 zeigt ein Flussdiagramm eines erfindungsgemäßen Verfahrens zum Liefern einer Information über einen Vitalparameter eines Lebewesens in einer Einrichtung mit einer Lichtquelle und einem Lichtempfänger, die angeordnet sind, um eine Information über eine optische Dämpfung in einem Körperteil des Lebewesens zwischen der Lichtquelle und dem Lichtempfänger zu bestimmen, wobei die optische Dämpfung von dem Vitalparameter abhängt, und wobei die Lichtquelle und der Lichtempfänger an dem Körperteil mit einer Befestigungseinrichtung angebracht sind. Das Verfahren gemäß der Fig. 7 ist in seiner Gesamtheit mit 700 bezeichnet. Das Verfahren 700 umfasst in einem ersten Schritt 710 ein Bestimmen einer Information über eine Beschleunigung der Lichtquelle, des Lichtempfängers oder der Befestigungseinrichtung. In einem zweiten Schritt 720 wird geprüft, ob die Beschleunigung größer oder kleiner als eine vorgegebene maximale Beschleunigung ist. Ist die Beschleunigung kleiner bzw. nicht größer als die vorgegebene maximale Beschleunigung, so wird in einem Schritt 730 eine Information über den Vitalparameter des Lebewesens aus einer Information über eine optische Dämpfung in dem Körperteil des Lebewesens zwischen dem Lichtsender und dem Lichtempfänger bestimmt. Wird hingegen in dem Schritt 720 festgestellt, dass die Beschleunigung größer als die vorgegebene maximale Beschleunigung ist, so wird der Lichtsender abgeschaltet und/oder die Erzeugung der Information über den Vitalparameter unter Verwendung der Information über die optische Dämpfung eingestellt bzw. unterbrochen.

Das Verfahren 700 kann ferner um all diejenigen Schritte ergänzt werden, die von der erfindungsgemäßen oben beschriebenen Vorrichtung durchgeführt werden.

Die erfindungsgemäßen Verfahren können in beliebiger Weise realisiert werden. Beispielsweise kann das erfindungsgemäße Verfahren durch eine elektronische Rechenanlage bzw. durch einen Computer realisiert werden.

In anderen Worten, die erfindungsgemäße Vorrichtung und das erfindungsgemäße Verfahren kann in Hardware oder in Software implementiert werden. Die Implementation kann auf einem digitalen Speichermedium, beispielsweise einer Diskette, einer CD, einer DVD, einem ROM-, PROM-, EPROM-, EEPROM- oder einem FLASH-Speicher mit elektronisch auslesbaren Steuersignalen erfolgen, die so mit einem programmierbaren Computersystem zusammenwirken können, dass das entsprechende Verfahren ausgeführt wird.

Allgemein besteht die vorliegende Erfindung somit auch in einem Computer-Programm-Produkt mit auf einem maschinenlesbaren Träger gespeicherten Programmcode zur Durchführung zumindest eines der erfindungsgemäßen Verfahren, wenn das Computerprogramm-Produkt auf einem Rechner abläuft. In anderen Worten ausgedrückt, kann die Erfindung somit als ein Computer-Programm mit einem Programmcode zur Durchführung eines erfindungsgemäßen Verfahrens realisiert werden, wenn das Computer-Programm auf einem Computer abläuft.

Zusammenfassend ist somit festzuhalten, dass die vorliegende Erfindung eine Sensorvorrichtung zur nichtinvasiven Aufnahme eines Plethysmogramms am Handgelenk eines Menschens umfasst. Gemäß einem Aspekt umfasst die vorliegende Erfindung eine gleichzeitige Messung der Bewegung der Sensorvorrichtung und der Hautimpedanz. Ein Plethysmogramm ist dabei eine graphische Abbildung von Volumenänderungen, z. B. von Arterien eines Lebewesen.

Die vorliegende Erfindung basiert unter Anderem auf der Erkenntnis, dass Photoplethysmographen (beispielsweise Photoplethysmographen für den Einsatz am Finger, die beispielsweise durch einen Fingerclip an der Fingerkuppe angebracht werden) sehr empfindlich auf Erschütterungen reagieren, was eine zuverlässige Aufnahme bzw. Auswertung des Plethysmogramms erheblich erschwert.

Der vorliegenden Erfindung liegt daher die Aufgabe zugrunde, ein Plethysmogramm am Handgelenk mit einer Sensorvorrichtung basierend auf dem Transmissionsprinzip zu erfassen. Die Sensorvorrichtung wird am Handgelenk getragen, um eine Einschränkung einer Bewegungsfreiheit eines Menschen auf ein Minimum zu reduzieren. Beschleunigungssensoren beispielsweise für die Koordinatenachsen in einem dreidimensionalen Raum detektieren Bewegungen und Erschütterungen der Sensorvorrichtung und ermöglichen eine nachträgliche Korrektur und eine Plausibilitätsbewertung eines eventuell durch Bewegungsartefakte gestörten Plethysmogramms. Mittels Elektroden wird eine Hautimpedanz am Handgelenk gemessen, und daraus der Wasseranteil in einem Gewebe, der im Wesentlichen zur Dämpfung des eingestrahlten Lichts beiträgt, bestimmt. Entsprechend dem Wasseranteil in dem Gewebe wird die (in das Gewebe) eingestrahlte Lichtleistung (bzw. Lichtenergie) optimal eingestellt. Somit wird eine Reduzierung des Energieverbrauchs (bezogen auf herkömmliche Plethysmographen, bei denen eine vorgegebene Lichtleistung bzw. Lichtenergie verwendet wird) erreicht. Des Weiteren werden (optional) zur Abschätzung von Schlaganfallrisiken aus dem Wasseranteil (im Gewebe) ein Austrocknungsgrad (Exsikkationsgrad) und eine Blutviskosität des Patienten ermittelt.

Der erfindungsgemäße Plethysmograph wird am Handgelenk ähnlich einer Armbanduhr getragen und nicht (wie herkömmlicherweise üblich) am Finger eines Patienten. Somit ist der Finger nicht blockiert, und der Patient nicht in seiner Bewegungsfreiheit eingeschränkt. Die erfindungsgemäß verwendeten Bewegungssensoren (bzw. Beschleunigungssensoren) erlauben eine Plausibilitätsbewertung und eine Korrektur eines eventuell durch Bewertungsartefakte gestörten Plethysmogramms. Das Plethysmogramm wird zuverlässiger aufgenommen, da der Plethysmograph unempfindlich gegenüber Erschütterungen ist. Durch Messung der Hautimpedanz kann über den Wasseranteil des Gewebes auf den Grad der Austrocknung (Exsikkation) geschlossen werden, und es können Schlaganfallrisiken abgeschätzt werden. Außerdem lässt sich in der erfindungsgemäßen Weise damit die eingestrahlte Lichtleistung optimal anpassen und der Energieverbrauch der Sensorvorrichtung minimieren.

Die vorliegende Erfindung schafft somit ganz allgemeine eine Vorrichtung zur bewegungsunempfindlichen bzw. erschütterungsunempfindlichen Bestimmung eines Vitalparameters basierend auf einem Lichtintensitätssignal, das durch eine Durchstrahlung bzw. Durchleuchtung eines Körperteils eines Patienten unter Verwendung einer an dem Patienten mit Hilfe einer Befestigungseinrichtung angebrachten Lichtquelle und eines Lichtempfängers erzeugt wird. Durch die Berücksichtigung von Bewegungen können präzisere Ergebnisse als mit herkömmlichen Messeinrichtungen erzielt werden, und es kann gleichzeitig eine maximale Bewegungsfreiheit eines Patienten bzw. Lebewesens auch während der Durchführung einer Messung sichergestellt werden.

## Patentansprüche

1. Sensor (100; 150; 300; 350; 410) zum Liefern einer Information (142; 720; 250; 280) über einen Vitalparameter eines Lebewesens, mit folgenden Merkmalen:
einer Befestigungseinrichtung (110; 1) zum Anbringen des Sensors an dem Lebewesen;
einer Lichtquelle (120; 2a), die mit der Befestigungseinrichtung verbunden ist, um Licht in ein Körperteil des Lebewesens einzustrahlen;
einem Lichtempfänger (124; 2b), der mit der Befestigungseinrichtung verbunden ist, und der ausgelegt ist, um einen Teil des eingestrahlten Lichts zu empfangen, und um in Abhängigkeit von einer Intensität des empfangenen Lichts ein Lichtintensitätssignal (126) zu liefern, das von dem Vitalparameter abhängt;
einem Beschleunigungssensor (130; 8), der mit der Befestigungseinrichtung verbunden ist, und der ausgelegt ist, um in Abhängigkeit von einer Beschleunigung in zumindest einer Richtung ein Beschleunigungssignal (136) zu liefern; und
einer Lichtquellen-Ansteuereinrichtung (420), die ausgelegt ist, um die Lichtquelle abzuschalten, wenn das Beschleunigungssignal anzeigt, dass die Beschleunigung größer als eine vorgegebene maximal zulässige Beschleunigung ist;
wobei der Sensor ausgelegt ist, um das Lichtintensitätssignal und das Beschleunigungssignal an eine Verarbeitungseinrichtung zur verknüpfenden Verarbeitung des Lichtintensitätssignals und des Beschleunigungssignals zu übertragen.

2. Sensor (100; 150; 300; 350; 410) gemäß Anspruch 1, wobei der Vitalparameter eine Pulsfrequenz eines Lebewesens umfasst, und wobei die Lichtquelle (120; 2a) und der Lichtempfänger (124; 2b) derart angeordnet sind, dass ein optische Dämpfung in dem Körperteil zwischen der Lichtquelle und dem Lichtempfänger durch eine Veränderung eines Volumens eines Blutgefäßes in dem Körperteil (210) beeinflusst wird.

3. Sensor (100; 150; 300; 350; 410) gemäß Anspruch 1 oder 2, wobei der Vitalparameter einen Anteil verschiedener Blutbestandteile von Blut in einem Blutgefäß des Lebewesens umfasst, wobei die Anteile der verschiedenen Blutbestandteile des Blutes eine WellenlängenAbhängigkeit einer optischen Dämpfung in dem Körperteil zwischen der Lichtquelle (120; 2a) und dem Lichtempfänger (124; 2b) beeinflussen, und
wobei der Sensor ausgelegt ist, um die Wellenlängenabhängigkeit der optischen Dämpfung in dem Körperteil zwischen der Lichtquelle und dem Lichtempfänger zu bestimmen.

4. Sensor (100; 150; 300; 350; 410) gemäß einem der Ansprüche 1 bis 3, bei dem die Lichtquelle (120; 2a) und der Lichtempfänger (124; 2b) angeordnet sind, um eine Transmissionsmessung durch das Körperteil (310) zu ermöglichen:

5. Sensor (100; 150; 300; 350; 410) gemäß einem der Ansprüche 1 bis 4, bei dem die Befestigungseinrichtung (110; 1) ausgelegt ist, um den Sensor um eine menschliche Handwurzel, ein menschliches Handgelenk oder einen menschlichen Unterarm anzubringen.

6. Sensor (100; 150; 300; 350; 410) gemäß Anspruch 5, bei der die Befestigungseinrichtung (110; 1) ausgelegt ist, um den Sensor um ein menschliches Handgelenk anzubringen, und bei dem die Lichtquelle (120; 2a) an der Befestigungseinrichtung angebracht ist, um von einer Außenseite des Handgelenks her Licht in das Handgelenk einzustrahlen.

7. Sensor (100; 150; 300; 350; 410) gemäß Anspruch 5 oder 6, bei der die Befestigungseinrichtung (110; 1) ausgelegt ist, um den Sensor um ein menschliches Handgelenk anzubringen, und bei dem der Lichtempfänger (124; 2b) an der Befestigungseinrichtung angebracht ist, um Licht von einer Innenseite des Handgelenks zu empfangen.

8. Sensor (100; 150; 300; 350; 410) gemäß einem der Ansprüche 1 bis 5, bei dem die Befestigungseinrichtung (110; 1) ausgelegt ist, um den Sensor um ein menschliches Sprungbein, ein menschliches Sprunggelenk oder einen menschlichen Unterschenkel anzubringen.

9. Sensor (100; 150; 300; 350; 410) gemäß einem der Ansprüche 1 bis 8, bei dem die Befestigungseinrichtung (110; 1), die Lichtquelle (120; 2a) und der Lichtempfänger (124; 2b) ausgelegt sind, um den Sensor so an einem Körperteil oder um ein Körperteil zu befestigen, das eine in dem Körperteil vorhandene Arterie zwischen der Lichtquelle und dem Lichtempfänger angeordnet ist.

10. Sensor (100; 150; 300; 350; 410) gemäß einem der Ansprüche 1 bis 9, wobei der Sensor als Verarbeitungseinrichtung (140; 210; 240; 270; 19) eine Pulsbestimmungseinrichtung umfasst, die ausgelegt ist, um aus zeitlichen Schwankungen des Lichtintensitätssignals (126) eine Pulsfrequenz des Lebewesens zu bestimmen, wobei die Pulsfrequenz des Lebewesens den Vitalparameter bildet.

11. Sensor (100; 150; 300; 350; 410) gemäß einem der Ansprüche 1 bis 10, wobei der Sensor eine Mehrzahl von Lichtquellen (120; 2a) unterschiedlicher Lichtwellenlänge umfasst, und ausgelegt ist, um Licht unterschiedlicher Wellenlänge in das Körperteil einzustrahlen, um eine Bestimmung einer optischen Dämpfung zwischen den Lichtquellen und dem Lichtempfänger (124; 2b) in Abhängigkeit von der Wellenlänge zu ermöglichen.

12. Sensor (100; 150; 300; 350; 410) gemäß einem der Ansprüche 1 bis 11, wobei der Sensor eine Mehrzahl von Lichtempfängern (124; 2b) unterschiedlicher spektraler Empfindlichkeit umfasst, um eine Bestimmung einer optischen Dämpfung zwischen der Lichtquelle (120; 2a) und den Lichtempfängern in Abhängigkeit von der Wellenlänge zu ermöglichen.

13. Sensor (100; 150; 300; 350; 410) gemäß einem der Ansprüche 1 bis 12, wobei der Sensor als Verarbeitungseinrichtung (140; 210; 240; 270; 19) eine Blutzusammensetzungs-Bestimmungseinrichtung umfasst, die ausgelegt ist, um an einer Wellenlängenabhängigkeit der optischen Dämpfung in dem Körperteil zwischen der Lichtquelle (120; 2a) und dem Lichtempfänger (124; 2b) Anteile von verschiedenen Blutbestandteilen von Blut in einem Blutgefäß (4, 5) des Lebewesens zu bestimmen.

14. Sensor (100; 150; 300; 350; 410) gemäß einem der Ansprüche 1 bis 13, wobei der Sensor die Verarbeitungseinrichtung (140; 210; 240; 270; 19) umfasst, und wobei die Verarbeitungseinrichtung ausgelegt ist, um das Lichtintensitätssignal (126) in Abhängigkeit von dem Beschleunigungssignal (136) zu korrigieren, um Veränderungen des Lichtintensitätssignals aufgrund der Beschleunigung entgegenzuwirken.

15. Sensor (100; 150; 300; 350; 410) gemäß einem der Ansprüche 1 bis 14, wobei der Sensor die Verarbeitungseinrichtung (140; 210; 240; 270; 19) umfasst, wobei die Verarbeitungseinrichtung ausgelegt ist, um die Information über den Vitalparameter aus dem Lichtintensitätssignal (126) zu bestimmen, und wobei die Verarbeitungseinrichtung ferner ausgelegt ist, um die Information (126) über den Vitalparameter in Abhängigkeit von dem Beschleunigungssignal (136) zu korrigieren, um Veränderung des Lichtintensitätssignals (126) aufgrund der Beschleunigung entgegenzuwirken.

16. Sensor (100; 150; 300; 350; 410) gemäß einem der Ansprüche 1 bis 15, wobei der Sensor die Verarbeitungseinrichtung (140; 210; 240; 270) umfasst,
wobei die Verarbeitungseinrichtung ausgelegt ist, um die Information (142; 220; 250; 280) über den Vitalparameter aus dem Lichtintensitätssignal (126) zu bestimmen und um aus dem Beschleunigungssignal (136) eine der Informationen über den Vitalparameter zugeordnete Zuverlässigkeitsinformation (274) zu erzeugen, die bei Vorliegen einer betragsmäßig kleinen Beschleunigung eine hohe Zuverlässigkeit der Information über den Vitalparameter anzeigt, und die bei Vorliegen einer betragsmäßig größeren Beschleunigung eine geringere Zuverlässigkeit der Information über den Vitalparameter anzeigt.

17. Sensor (100; 150; 300; 350; 410) gemäß einem der Ansprüche 1 bis 16, wobei der Sensor die Verarbeitungseinrichtung (140; 210; 240; 270; 19) umfasst, und wobei die Verarbeitungseinrichtung ausgelegt ist, um die Information (142; 220; 250; 280) über den Vitalparameter aus dem Lichtintensitätssignal (126) nur dann zu bestimmen, oder auszugeben, wenn das Beschleunigungssignal (136) anzeigt, dass die Beschleunigung innerhalb eines vorgegebenen zulässigen Bereichs liegt, und um anderenfalls anstelle einer aktuellen Information über den Vitalparameter eine früher bestimmte Information über den Vitalparameter oder ein Fehlersignal, das einen Fehler anzeigt, zu liefern.

18. Sensor (100; 150; 300; 350; 410) gemäß einem der Ansprüche 1 bis 16, wobei der Sensor die Verarbeitungseinrichtung (140; 210; 240; 270; 410) umfasst, wobei die Verarbeitungseinrichtung ausgelegt ist, um die Informationen (142; 220; 250; 280) über den Vitalparameter aus dem Lichtintensitätssignal (126) nur dann zu bestimmen, wenn das Beschleunigungssignal (136) anzeigt, dass die Beschleunigung innerhalb eines vorgegebenen zulässigen Bereichs liegt, und um andernfalls keine Information über den Vitalparameter zu liefern.

19. Sensor (100; 150; 300; 350; 410) gemäß einem der Ansprüche 1 bis 18, wobei der Sensor ferner folgende Merkmale aufweist:
eine Dämpfungsmaß-Erfassungseinrichtung (120), die ausgelegt ist, um eine Dämpfungsinformation, die eine optische Dämpfung zwischen der Lichtquelle (120; 2a) und dem Lichtempfänger (124; 2b) beschreibt, zu ermitteln; und
eine Lichtquellen-Einstellungseinrichtung (510), die ausgelegt ist, um eine von der Lichtquelle eingestrahlte Lichtleistung oder Lichtenergie in Abhängigkeit von der Dämpfungsinformation zu bestimmen.

20. Sensor (100; 150; 300; 350; 410) gemäß Anspruch 19, bei der die Dämpfungsmaß-Erfassungseinrichtung (120) ausgelegt ist, um eine Information über einen Wasseranteil in einem Gewebe des Körperteils zu bestimmen, und um die Dämpfungsinformation von der Information über den Wasseranteil abzuleiten.

21. Sensor (100; 150; 300; 350; 410) gemäß Anspruch 19 oder 20, bei dem die Dämpfungsmaß-Erfassungseinrichtung (21) ausgelegt ist, um eine Hautimpedanz des Körperteils zu bestimmen, und um die Dämpfungsinformation von der Hautimpedanz abzuleiten.

22. Sensor (100; 150; 300; 350; 410) gemäß einem der Ansprüche 19 bis 21, bei dem die Befestigungseinrichtung (110; 1) eine erste Elektrode (20) und eine zweite Elektrode (20) umfasst, die angeordnet sind, um das Körperteil elektrisch zu kontaktieren, wobei die Dämpfungsmaß-Erfassungseinrichtung (21) ausgelegt ist, um eine Impedanz zwischen der ersten Elektrode und der zweiten Elektrode zu bestimmen, und um die Dämpfungsinformation von der Impedanz abzuleiten.

23. Sensor (100; 150; 300; 350; 410) zum Liefern einer Information (142; 720; 250; 280) über einen Vitalparameter eines Lebewesens, mit folgenden Merkmalen:
einer Befestigungseinrichtung (110; 1) zum Anbringen des Sensors an dem Lebewesen;
einer Lichtquelle (120; 2a), die mit der Befestigungseinrichtung verbunden ist, um Licht in ein Körperteil des Lebewesens einzustrahlen;
einem Lichtempfänger (124; 2b), der mit der Befestigungseinrichtung verbunden ist, und der ausgelegt ist, um einen Teil des eingestrahlten Lichts zu empfangen, und um in Abhängigkeit von einer Intensität des empfangenen Lichts ein Lichtintensitätssignal 126 zu liefern, das von dem Vitalparameter abhängt;
einem Beschleunigungssensor (130; 8), der mit der Befestigungseinrichtung verbunden ist, und der ausgelegt ist, um in Abhängigkeit von einer Beschleunigung in zumindest einer Richtung ein Beschleunigungssignal 136 zu liefern; und
einer verarbeitungseinrichtung (140, 240);
wobei der Sensor ausgelegt ist, um das Lichtintensitätssignal und das Beschleunigungssignal an die Verarbeitungseinrichtung zur verknüpfenden Verarbeitung des Lichtintensitätssignals und des Beschleunigungssignals zu übertragen,
wobei die Verarbeitungseinrichtung (140, 240) ausgelegt ist, um bei der verknüpfenden Verarbeitung die Information (142; 220; 250; 280) über den Vitalparameter aus dem Lichtintensitätssignal (126) nur dann zu bestimmen, oder auszugeben, wenn das Beschleunigungssignal (136) anzeigt, dass die Beschleunigung innerhalb eines vorgegebenen zulässigen Bereichs liegt, und um anderenfalls anstelle einer aktuellen Information über den Vitalparameter eine früher bestimmte Information über den Vitalparameter zu liefern.

24. Verfahren (700) zum Liefern einer Information (142; 220; 250; 280) über einen Vitalparameter eines Lebewesens in Verbindung mit einer Einrichtung mit einer Lichtquelle (120; 2a) und einem Lichtempfänger (124; 2b), die angeordnet sind, um eine Information über eine optische Dämpfung in einem Körperteil des Lebewesens zwischen dem Lichtsender und dem Lichtempfänger zu bestimmen, wobei die optische Dämpfung von dem Vitalparameter abhängt, und wobei die Lichtquelle und der Lichtempfänger an dem Körperteil mit einer Befestigungseinrichtung (110; 1) angebracht sind, mit folgenden Schritten:
Bestimmen (710) einer Information (126) über eine Beschleunigung der Lichtquelle, des Lichtempfängers oder der Befestigungseinrichtung; und
falls die Information über die Beschleunigung anzeigt, dass die Beschleunigung größer als eine vorgegeben maximal zulässige Beschleunigung ist, Abschalten (740) der Lichtquelle;
andernfalls Bestimmen der Information über den Vitalparameter des Lebewesens aus der Information über die optische Dämpfung in dem Körperteil des Lebewesens zwischen der Lichtquelle und dem Lichtempfänger und unter Verwendung des Beschleunigungssignals.

25. Verfahren (700) zum Liefern einer Information (142; 220; 250; 280) über einen Vitalparameter eines Lebewesens in Verbindung mit einer Einrichtung mit einer Lichtquelle (120; 2a) und einem Lichtempfänger (124; 2b), die angeordnet sind, um eine Information über eine optische Dämpfung in einem Körperteil des Lebewesens zwischen dem Lichtsender und dem Lichtempfänger zu bestimmen, wobei die optische Dämpfung von dem Vitalparameter abhängt, und wobei die Lichtquelle und der Lichtempfänger an dem Körperteil mit einer Befestigungseinrichtung (110; 1) angebracht sind, mit folgenden Schritten:
Bestimmen (710) einer Information (126) über eine Beschleunigung der Lichtquelle, des Lichtempfängers oder der Befestigungseinrichtung; und
falls die Information über die Beschleunigung anzeigt, dass die Beschleunigung größer als eine vorgegebene maximal zulässige Beschleunigung ist, Unterbrechen einer Erzeugung der Information über den Vitalparameter unter Verwendung der Information über die optische Dämpfung;
andernfalls Bestimmen der Information über den Vitalparameter des Lebewesens aus der Information über die optische Dämpfung in dem Körperteil des Lebewesens zwischen der Lichtquelle und dem Lichtempfänger und unter Verwendung des Beschleunigungssignals.

26. Verfahren gemäß Anspruch 25, wobei das Verfahren folgenden Schritt umfasst:
falls die Information über die Beschleunigung anzeigt, dass die Beschleunigung größer als eine vorgegebene maximal zulässige Beschleunigung ist, Liefern einer früher bestimmten Information über den Vitalparameter anstelle einer aktuellen Information über den Vitalparameter.

27. Verfahren gemäß Anspruch 25, wobei das Verfahren folgenden Schritt umfasst:
falls die Information über die Beschleunigung anzeigt, dass die Beschleunigung größer als eine vorgegebene maximal zulässige Beschleunigung ist, Liefern eines Fehlersignals, das einen Fehler anzeigt, anstelle einer aktuellen Information über den Vitalparameter.

28. Verfahren gemäß Anspruch 25, wobei das Verfahren folgenden Schritt umfasst:
falls die Information über die Beschleunigung anzeigt, dass die Beschleunigung größer als eine vorgegebene maximal zulässige Beschleunigung ist, Liefern keiner Information über den Vitalparameter.

29. Computerprogramm zur Durchführung des Verfahrens gemäß einem der Ansprüche 24 bis 28, wenn das Computerprogramm auf einem Computer abläuft.

## Claims

1. A sensor (100; 150; 300; 350; 410) for providing information (142; 720; 250; 280) on a vital parameter of a living being, comprising:
mounting means (110; 1) for attaching the sensor to the living being;
a light source (120; 2a) connected to the mounting means for radiating light into a part of the body of the living being;
a light receiver (124; 2b) connected to the mounting means and implemented to receive part of the light radiated to provide, in dependence on an intensity of the light received, a light intensity signal (126) depending on the vital parameter;
an acceleration sensor (130; 8) connected to the mounting means and implemented to provide an acceleration signal (136) in dependence on an acceleration in at least one direction; and
light source driving means (420) implemented to switch off the light source when the acceleration signal indicates that the acceleration is greater than a predetermined maximally allowed acceleration;
wherein the sensor is implemented to transfer the light intensity signal and the acceleration signal to processing means for a combining processing of the light intensity signal and the acceleration signal.

2. The sensor (100; 150; 300; 350; 410) according to claim 1, wherein the vital parameter includes a pulse frequency of a living being, and wherein the light source (120; 2a) and the light receiver (124; 2b) are arranged such that an optical attenuation in the part of the body between the light source and the light receiver is influenced by a change in volume in a blood vessel in the part of the body (210).

3. The sensor (100; 150; 300; 350; 410) according to claim 1 or 2, wherein the vital parameter includes a portion of different blood components of blood in a blood vessel of the living being, wherein the portions of the different blood components of the blood influence a wavelength dependence of an optical attenuation in the part of the body between the light source (120; 2a) and the light receiver (124; 2b), and
wherein the sensor is implemented to determine the wavelength dependence of the optical attenuation in the part of the body between the light source and the light receiver.

4. The sensor (100; 150; 300; 350; 410) according to one of claims 1 to 3, wherein the light source (120; 2a) and the light receiver (124; 2b) are arranged to allow transmission measurement through the part of the body (310).

5. The sensor (100; 150; 300; 350; 410) according to one of claims 1 to 4, wherein the mounting means (110; 1) is implemented to attach the sensor around a human carpus, a human wrist or a human forearm.

6. The sensor (100; 150; 300; 350; 410) according to claim 5, wherein the mounting means (110; 1) is implemented to attach the sensor to a human wrist, and wherein the light source (120; 2a) is attached to the mounting means to radiate light into the wrist from an outward side of the wrist.

7. The sensor (100; 150; 300; 350; 410) according to claim 5 or 6, wherein the mounting means (110; 1) is implemented to attach the sensor around a human wrist, and wherein the light receiver (124; 2b) is attached to the mounting means to receive light from an inward side of the wrist.

8. The sensor (100; 150; 300; 350; 410) according to one of claims 1 to 5, wherein the mounting means (110; 1) is implemented to attach the sensor around a human ankle bone, a human ankle joint or a human lower leg.

9. The sensor (100; 150; 300; 350; 410) according to one of claims 1 to 8, wherein the mounting means (110; 1), the light source (120; 2a) and the light receiver (124; 2b) are implemented to mount the sensor to a part of the body or around a part of the body such that an artery in the part of the body is arranged between the light source and the light receiver.

10. The sensor (100; 150; 300; 350; 410) according to one of claims 1 to 9, wherein the sensor includes, as processing means (140; 210; 240; 270; 19), pulse determining means implemented to determine a pulse frequency of the living being from temporal variations of the light intensity signal (126), the pulse frequency of the living being representing the vital parameter.

11. The sensor (100; 150; 300; 350; 410) according to one of claims 1 to 10, wherein the sensor includes a plurality of light sources (120; 2a) of different light wavelengths and is implemented to radiate light of different wavelengths into the part of the body to allow determining an optical attenuation between the light sources and the light receiver (124; 2b) in dependence on the wavelength.

12. The sensor (100; 150; 300; 350; 410) according to one of claims 1 to 11, wherein the sensor includes a plurality of light receivers (124; 2b) of different spectral sensitivities to allow determining an optical attenuation between the light source (120; 2a) and the light receivers in dependence on the wavelength.

13. The sensor (100; 150; 300; 350; 410) according to one of claims 1 to 12, wherein the sensor includes, as processing means (140; 210; 240; 270; 19), blood composition determining means implemented to determine, using a wavelength dependence of the optical attenuation in the part of the body between the light source (120; 2a) and the light receiver (124; 2b), portions of different blood components of blood in a blood vessel (4, 5) of the living being.

14. The sensor (100; 150; 300; 350; 410) according to one of claims 1 to 13, wherein the sensor includes the processing means (140; 210; 240; 270; 19), and wherein the processing means is implemented to correct the light intensity signal (126) in dependence on the acceleration signal (136) to counteract changes in the light intensity signal due to the acceleration.

15. The sensor (100; 150; 300; 350; 410) according to one of claims 1 to 14, wherein the sensor includes the processing means (140; 210; 240; 270; 19), wherein the processing means is implemented to determine the information on the vital parameter from the light intensity signal (126), and wherein the processing means is additionally implemented to correct the information (126) on the vital parameter in dependence on the acceleration signal (136) to counteract a change in the light intensity signal (126) due to the acceleration.

16. The sensor (100; 150; 300; 350; 410) according to one of claims 1 to 15, wherein the sensor includes the processing means (140; 210; 240; 270), wherein the processing means is implemented to determine the information (142; 220; 250; 280) on the vital parameter from the light intensity signal (126) and to generate from the acceleration signal (136) reliability information (274) associated to the information on the vital parameter which indicates high reliability of the information on the vital parameter with small an acceleration magnitude and indicates lower a reliability of the information on the vital parameter with greater an acceleration magnitude.

17. The sensor (100; 150; 300; 350; 410) according to one of claims 1 to 16, wherein the sensor includes processing means (140; 210; 240; 270; 19), and wherein the processing means is implemented to only determine the information (142; 220; 250; 280) on the vital parameter from the light intensity signal (126) or output same if the acceleration signal (136) indicates that the acceleration is within a predetermined allowed region, and to otherwise provide, instead of current information on the vital parameter, information, determined before, on the vital parameter or an error signal indicating an error.

18. The sensor (100; 150; 300; 350; 410) according to one of claims 1 to 16, wherein the sensor includes the processing means (140; 210; 240; 270; 410), wherein the processing means is implemented to only determine the information (142; 220; 250; 280) on the vital parameter from the light intensity signal (126) if the acceleration signal (136) indicates that the acceleration is within a predetermined allowed region, and otherwise not to provide information on the vital parameter.

19. The sensor (100; 150; 300; 350; 410) according to one of claims 1 to 18, the sensor further comprising:
attenuation measure detecting means (120) implemented to determine attenuation information describing an optical attenuation between the light source (120; 2a) and the light receiver (124; 2b); and
light source adjusting means (501) implemented to determine a light power or light energy radiated by the light source in dependence on the attenuation information.

20. The sensor (100; 150; 300; 350; 410) according to claim 19, wherein the attenuation measure detecting means (120) is implemented to determine information on a water portion in a tissue of the part of the body and to derive the attenuation information from the information on the water portion.

21. The sensor (100; 150; 300; 350; 410) according to claim 19 or 20, wherein the attenuation measure detecting means (21) is implemented to determine a skin impedance of the part of the body and to derive the attenuation information from the skin impedance.

22. The sensor (100; 150; 300; 350; 410) according to one of claims 19 to 21, wherein the mounting means (110; 1) includes a first electrode (20) and a second electrode (20) which are arranged to electrically contact the part of the body, the attenuation measure detecting means (21) being implemented to determine an impedance between the first electrode and the second electrode and to derive the attenuation information from the impedance.

23. A sensor (100; 150; 300; 350; 410) for providing information (142; 720; 250; 280) on a vital parameter of a living being, comprising:
mounting means (110; 1) for attaching the sensor to the living being;
a light source (120; 2a) connected to the mounting means for radiating light into a part of the body of the living being;
a light receiver (124; 2b) connected to the mounting means and implemented to receive part of the light radiated to provide, in dependence on an intensity of the light received, a light intensity signal (126) depending on the vital parameter;
an acceleration sensor (130; 8) connected to the mounting means and implemented to provide an acceleration signal (136) in dependence on an acceleration in at least one direction; and
processing means (140, 240),
wherein the sensor is implemented to transfer the light intensity signal and the acceleration signal to the processing means for a combining processing of the light intensity signal and the acceleration signal,
wherein the processing means (140, 240) is implemented to only determine the information (142; 720; 250; 280) on the vital parameter from the light intensity signal (126) or output same if the acceleration signal (136) indicates that the acceleration is within a predetermined allowed region, and to otherwise provide, instead of current information on the vital parameter, information, determined before, on the vital parameter.

24. A method (700) for providing information (142; 220; 250; 280) on a vital parameter of a living being in connection with means having a light source (120; 2a) and a light receiver (124; 2b) which are arranged to determine information on an optical attenuation in a part of the body of the living being between the light transmitter and the light receiver, the optical attenuation depending on the vital parameter, and the light source and the light receiver being attached to the part of the body by mounting means (110; 1), comprising the steps of:
determining (710) information (126) on an acceleration of the light source, the light receiver or the mounting means; and
should the information on the acceleration indicate that the acceleration is greater than a predetermined maximally allowed acceleration, switching off (740) the light source;
otherwise determining the information on the vital parameter of the living being from the information on the optical attenuation in the part of the body of the living being between the light source and the light receiver using the acceleration signal.

25. A method (700) for providing information (142; 220; 250; 280) on a vital parameter of a living being in connection with means having a light source (120; 2a) and a light receiver (124; 2b) which are arranged to determine information on an optical attenuation in a part of the body of the living being between the light transmitter and the light receiver, the optical attenuation depending on the vital parameter, and the light source and the light receiver being attached to the part of the body by mounting means (110; 1), comprising the steps of:
determining (710) information (126) on an acceleration of the light source, the light receiver or the mounting means; and
should the information on the acceleration indicate that the acceleration is greater than a predetermined maximally allowed acceleration, interrupting a generation of the information on the vital parameter using the information on the optical attenuation;
otherwise determining the information on the vital parameter of the living being from the information on the optical attenuation in the part of the body of the living being between the light source and the light receiver using the acceleration signal.

26. The method according to claim 25, the method comprising the step of:
should the information on the acceleration indicate that the acceleration is greater than a predetermined maximally allowed acceleration, providing, instead of current information on the vital parameter, information, determined before, on the vital parameter.

27. The method according to claim 25, the method comprising the step of:
should the information on the acceleration indicate that the acceleration is greater than a predetermined maximally allowed acceleration, providing, instead of current information on the vital parameter, an error signal indicating an error.

28. The method according to claim 25, the method comprising the step of:
should the information on the acceleration indicate that the acceleration is greater than a predetermined maximally allowed acceleration, providing no information on the vital parameter.

29. A computer program for performing the method according to one of claims 24 to 28 when the computer program runs on a computer.

## Revendications

1. Capteur (100; 150; 300; 350; 410) pour fournir une information (142; 720; 250; 280) sur un paramètre vital d'un être vivant, aux caractéristiques suivantes:
un moyen de fixation (110; 1) pour placer le capteur sur l'être vivant;
une source de lumière (120; 2a) qui est reliée au moyen de fixation, pour rayonner de la lumière dans une partie de corps de l'être vivant;
un récepteur de lumière (124; 2b) qui est relié au moyen de fixation et qui est conçu pour recevoir une partie de la lumière rayonnée, et pour fournir, en fonction d'une intensité de la lumière reçue, un signal d'intensité de lumière (126) qui dépend du paramètre vital;
un capteur d'accélération (130; 8) qui est relié au moyen de fixation et qui est conçu pour fournir, en fonction d'une accélération dans au moins une direction, un signal d'accélération (136); et
un moyen d'activation de source de lumière (420) qui est conçu pour couper la source de lumière lorsque le signal d'accélération indique que l'accélération est supérieure à une accélération admissible maximale prédéterminée;
le capteur étant conçu pour transmettre le signal d'intensité de lumière et le signal d'accélération à un dispositif de traitement pour le traitement de couplage du signal d'intensité de lumière et du signal d'accélération.

2. Capteur (100; 150; 300; 350; 410) selon la revendication 1, dans lequel le paramètre vital comprend une fréquence de pouls d'un être vivant, et dans lequel la source de lumière (120; 2a) et le récepteur de lumière (124; 2b) sont disposés de sorte qu'une atténuation optique dans la partie de corps entre la source de lumière et le récepteur de lumière soit influencée par une variation d'un volume d'un vaisseau sanguin dans la partie de corps (210).

3. Capteur (100; 150; 300; 350; 410) selon la revendication 1 ou 2, dans lequel le paramètre vital comprend une part de différents composants du sang dans un vaisseau sanguin de l'être vivant, les parts des différents composants du sang ayant une influence sur la dépendance de la longueur d'onde d'une atténuation optique dans la partie de corps entre la source de lumière (120; 2a) et le récepteur de lumière (124; 2b), et
dans lequel le capteur est conçu pour déterminer la dépendance de la longueur d'onde de l'atténuation optique dans la partie de corps entre la source de lumière et le récepteur de lumière.

4. Capteur (100; 150; 300; 350; 410) selon l'une des revendications 1 à 3, dans lequel la source de lumière (120; 2a) et le récepteur de lumière (124; 2b) sont disposés pour permettre une mesure de transmission par la partie de corps (310).

5. Capteur (100; 150; 300; 350; 410) selon l'une des revendications 1 à 4, dans lequel le moyen de fixation (110; 1) est conçu pour placer le capteur autour d'un carpe humain, d'un poignet humain ou d'un avant-bras humain.

6. Capteur (100; 150; 300; 350; 410) selon la revendication 5, dans lequel le moyen de fixation (110; 1) est conçu pour placer le capteur autour d'un poignet humain, et dans lequel la source de lumière (120; 2a) est placée sur le moyen de fixation, pour rayonner depuis un côté extérieur du poignet de la lumière dans le poignet.

7. Capteur (100; 150; 300; 350; 410) selon la revendication 5 ou 6, dans lequel le moyen de fixation (110; 1) est conçu pour placer le capteur autour d'un poignet humain, et dans lequel le récepteur de lumière (124; 2b) est placé sur le moyen de fixation, pour recevoir de la lumière d'un côté intérieur du poignet.

8. Capteur (100; 150; 300; 350; 410) selon l'une des revendications 1 à 5, dans lequel le moyen de fixation (110; 1) est conçu pour placer le capteur autour d'un astragale humain, d'un jarret humain ou d'une jambe humaine.

9. Capteur (100; 150; 300; 350; 410) selon l'une des revendications 1 à 8, dans lequel le moyen de fixation (110; 1), la source de lumière (120; 2a) et le récepteur de lumière (124; 2b) sont conçus pour fixer le capteur à une partie de corps ou autour d'une partie de corps de sorte qu'une artère présente dans la partie de corps soit disposée entre la source de lumière et le récepteur de lumière.

10. Capteur (100; 150; 300; 350; 410) selon l'une des revendications 1 à 9, dans lequel le capteur comprend, comme moyen de traitement (140; 210; 240; 270; 19), un moyen de détermination de pouls qui est conçu pour déterminer, à partir des variations dans le temps du signal d'intensité de lumière (126), une fréquence de pouls de l'être vivant, la fréquence de pouls de l'être vivant constituant le paramètre vital.

11. Capteur (100; 150; 300; 350; 410) selon l'une des revendications 1 à 10, dans lequel le capteur comporte une pluralité de sources de lumière (120; 2a) de différente longueur d'onde de lumière et est conçu pour rayonner de la lumière de longueur d'onde différente dans la partie de corps, pour permettre une détermination d'une atténuation optique entre les sources de lumière et le récepteur de lumière (124; 2b) en fonction de la longueur d'onde.

12. Capteur (100; 150; 300; 350; 410) selon l'une des revendications 1 à 11, dans lequel le capteur comporte une pluralité de récepteurs de lumière (124; 2b) de différente sensibilité spectrale, pour permettre une détermination d'une atténuation optique entre la source de lumière (120; 2a) et les récepteur de lumière en fonction de la longueur d'onde.

13. Capteur (100; 150; 300; 350; 410) selon l'une des revendications 1 à 12, dans lequel le capteur comprend, comme moyen de traitement (140; 210; 240; 270; 19), un moyen de détermination de composition de sang qui est conçu pour déterminer selon une dépendance de la longueur d'onde de l'atténuation optique dans la partie de corps entre la source de lumière (120; 2a) et le récepteur de lumière (124; 2b), les parts de différents composants du sang dans un vaisseau sanguin (4, 5) de l'être vivant.

14. Capteur (100; 150; 300; 350; 410) selon l'une des revendications 1 à 13, dans lequel le capteur comporte le moyen de traitement (140; 210; 240; 270; 19), et dans lequel le dispositif de traitement est conçu pour corriger le signal d'intensité de lumière (126) en fonction du signal d'accélération (136), pour contrecarrer les variations du signal d'intensité de lumière sur base de l'accélération.

15. Capteur (100; 150; 300; 350; 410) selon l'une des revendications 1 à 14, dans lequel le capteur comporte le moyen de traitement (140; 210; 240; 270; 19), le dispositif de traitement étant conçu pour déterminer l'information sur le paramètre vital à partir du signal d'intensité de lumière (126), et le dispositif de traitement étant par ailleurs conçu pour corriger l'information (126) sur le paramètre vital en fonction du signal d'accélération (136), pour contrecarrer une variation du signal d'intensité de lumière (126) sur base de l'accélération.

16. Capteur (100; 150; 300; 350; 410) selon l'une des revendications 1 à 15, le capteur comportant le moyen de traitement (140; 210; 240; 270),
dans lequel le dispositif de traitement est conçu pour déterminer l'information (142; 220; 250; 280) sur le paramètre vital à partir du signal d'intensité de lumière (126) et pour générer, à partir du signal d'accélération (136), une information de fiabilité (274) associée à l'information sur le paramètre vital qui, en cas de présence d'une accélération de faible quantité, indique une haute fiabilité de l'information sur le paramètre vital et qui, en cas de présence d'une accélération d'une quantité supérieure, indique une moindre fiabilité de l'information sur le paramètre vital.

17. Capteur (100; 150; 300; 350; 410) selon l'une des revendications 1 à 16, dans lequel le capteur comporte le dispositif de traitement (140; 210; 240; 270; 19), et dans lequel le dispositif de traitement est conçu pour ne déterminer, ou sortir, l'information (142; 220; 250; 280) sur le paramètre vital à partir du signal d'intensité de lumière (126) que lorsque le signal d'accélération (136) indique que l'accélération se situe dans une plage admissible prédéterminée,
et pour fournir autrement, au lieu d'une information actuelle sur le paramètre vital, une information déterminée antérieurement sur le paramètre vital ou un signal d'erreur qui indique une erreur.

18. Capteur (100; 150; 300; 350; 410) selon l'une des revendications 1 à 16, dans lequel le capteur comporte le moyen de traitement (140; 210; 240; 270; 410), dans lequel le dispositif de traitement est conçu pour ne déterminer les informations (142; 220; 250; 280) sur le paramètre vital à partir du signal d'intensité de lumière (126) que lorsque le signal d'accélération (136) indique que l'accélération se situe dans une plage admissible prédéterminée, et pour autrement ne fournir aucune information sur le paramètre vital.

19. Capteur (100; 150; 300; 350; 410) selon l'une des revendications 1 à 18, dans lequel le capteur présente par ailleurs les caractéristiques suivantes:
un moyen de saisie de mesure d'atténuation (120) qui est conçu pour déterminer une information d'atténuation qui décrit une atténuation optique entre la source de lumière (120; 2a) et le récepteur de lumière (124; 2b); et
un moyen de réglage de source de lumière (510) qui est conçu pour déterminer une puissance de lumière ou énergie de lumière rayonnée par la source de lumière en fonction de l'information d'atténuation.

20. Capteur (100; 150; 300; 350; 410) selon la revendication 19, dans lequel le moyen de saisie de mesure d'atténuation (120) est conçu pour déterminer une information sur une part d'eau dans un tissu de la partie de corps, et pour dériver l'information d'atténuation de l'information sur la part d'eau.

21. Capteur (100; 150; 300; 350; 410) selon la revendication 19 ou 20, dans lequel le moyen de saisie de mesure d'atténuation (21) est conçu pour déterminer une impédance de peau de la partie de corps, et pour dériver l'information d'atténuation de l'impédance de peau.

22. Capteur (100; 150; 300; 350; 410) selon l'une des revendications 19 à 21, dans lequel le moyen de fixation (110; 1) comporte une première électrode (20) et une deuxième électrode (20) qui sont disposées de manière à entrer en contact électrique avec la partie de corps, dans lequel le moyen de saisie de mesure d'atténuation (21) est conçu pour déterminer une impédance entre la première électrode et la deuxième électrode, et pour dériver l'information d'atténuation de l'impédance.

23. Capteur (100; 150; 300; 350; 410) pour fournir une information (142; 720; 250; 280) sur un paramètre vital d'un être vivant, aux caractéristiques suivantes:
un moyen de fixation (110; 1) destiné à placer le capteur sur l'être vivant;
une source de lumière (120; 2a) qui est reliée au moyen de fixation, pour rayonner de la lumière dans une partie de corps de l'être vivant;
un récepteur de lumière (124; 2b) qui est relié au moyen de fixation, et qui est conçu pour recevoir une partie de la lumière rayonnée, et pour fournir, en fonction d'une intensité de la lumière reçue, un signal d'intensité de lumière 126 qui dépend du paramètre vital;
un capteur d'accélération (130; 8) qui est relié au moyen de fixation et qui est conçu pour fournir, en fonction d'une accélération dans au moins une direction, un signal d'accélération 136; et
un dispositif de traitement (140, 240);
dans lequel le capteur est conçu pour transmettre le signal d'intensité de lumière et le signal d'accélération au dispositif de traitement pour le traitement de couplage du signal d'intensité de lumière et du signal d'accélération,
dans lequel le dispositif de traitement (140, 240) est conçu pour ne déterminer, ou sortir, lors du traitement de couplage, l'information (142; 220; 250; 280) sur le paramètre vital à partir du signal d'intensité de lumière (126) que lorsque le signal d'accélération (136) indique que l'accélération se situe dans une plage admissible prédéterminée, et pour fournir autrement, au lieu d'une information actuelle sur le paramètre vital, une information sur le paramètre vital déterminée antérieurement.

24. Procédé (700) pour fournir une information (142; 220; 250; 280) sur un paramètre vital d'un être vivant en rapport avec un moyen avec une source de lumière (120; 2a) et un récepteur de lumière (124; 2b) qui sont disposés de manière à déterminer une information sur une atténuation optique dans une partie de corps de l'être vivant entre l'émetteur de lumière et le récepteur de lumière, l'atténuation optique dépendant du paramètre vital, et la source de lumière et le récepteur de lumière étant placés sur la partie de corps par un moyen de fixation (110; 1), aux étapes suivantes consistant à:
déterminer (710) une information (126) sur une accélération de la source de lumière, du récepteur de lumière ou du moyen de fixation; et
si l'information sur l'accélération indique que l'accélération est supérieure à une accélération admissible maximale prédéterminée, couper (740) la source de lumière;
autrement, déterminer l'information sur le paramètre vital de l'être vivant à partir de l'information sur l'atténuation optique dans la partie de corps de l'être vivant entre la source de lumière et le récepteur de lumière et à l'aide du signal d'accélération.

25. Procédé (700) pour fournir une information (142; 220; 250; 280) sur un paramètre vital d'un être vivant en rapport avec un moyen avec une source de lumière (120; 2a) et un récepteur de lumière (124; 2b) qui sont disposés de manière à déterminer une information sur une atténuation optique dans une partie de corps de l'être vivant entre l'émetteur de lumière et le récepteur de lumière, l'atténuation optique dépendant du paramètre vital, et la source de lumière et le récepteur de lumière étant placés sur la partie de corps par un moyen de fixation (110; 1), aux étapes suivantes consistant à:
déterminer (710) une information (126) sur une accélération de la source de lumière, du récepteur de lumière ou du moyen de fixation; et
au cas où l'information sur l'accélération indique que l'accélération est supérieure à une accélération admissible maximale prédéterminée, interrompre une génération de l'information sur le paramètre vital à l'aide de l'information sur l'atténuation optique;
autrement, déterminer l'information sur le paramètre vital de l'être vivant à partir de l'information sur l'atténuation optique dans la partie de corps de l'être vivant entre la source de lumière et le récepteur de lumière et à l'aide du signal d'accélération.

26. Procédé selon la revendication 25, dans lequel le procédé comprend l'étape suivante consistant à:
au cas où l'information sur l'accélération indique que l'accélération est supérieure à une accélération admissible maximale prédéterminée, fournir une information sur le paramètre vital déterminé antérieurement au lieu d'une information actuelle sur le paramètre vital.

27. Procédé selon la revendication 25, dans lequel le procédé comprend l'étape suivante consistant à:
au cas où l'information sur l'accélération indique que l'accélération est supérieure à une accélération admissible maximale prédéterminée, fournir un signal d'erreur qui indique une erreur, au lieu d'une information actuelle sur le paramètre vital.

28. Procédé selon la revendication 25, dans lequel le procédé comprend l'étape suivante consistant à:
au cas où l'information sur l'accélération indique que l'accélération est supérieure à une accélération admissible maximale prédéterminée, ne pas fournir d'information sur le paramètre vital.

29. Programme d'ordinateur pour réaliser le procédé selon l'une des revendications 24 à 28 lorsque le programme d'ordinateur est exécuté sur un ordinateur.
